# FASCICULE DE BREVET EUROPEEN

(11) **EP 1 144 421 B1**
(45) Date de publication et mention de la délivrance du brevet: **08.10.2003**
(21) Numéro de dépôt: 00900598.4
(22) Date de dépôt: 18.01.2000
(51) Int. Cl.: C07F 9/30, A61K 31/66, C07F 9/572, C07F 9/6558, A61P 35/00

(54) **PSEUDO-PEPTIDES PHOSPHINIQUES INHIBITEURS DES METALLOPROTEASES MATRICIELLES**
PHOSPHINIC PSEUDOPEPTIDE ALS MATRIX METALLOPROTEASE-INHIBITOREN
PHOSPHINIC PSEUDOPEPTIDES INHIBITORS OF MATRIX METALLOPROTEASES

(30) Priorité: 19.01.1999 FR 9900509
(43) Date de publication de la demande: 17.10.2001
(73) Titulaire: COMMISSARIAT A L'ENERGIE ATOMIQUE, 75752 Paris Cédex 15 (FR); INSTITUT NATIONAL DE LA SANTE ET DE LA RECHERCHE MEDICALE (INSERM), 75654 Paris Cédex 13 (FR); CENTRE NATIONAL DE LA RECHERCHE SCIENTIFIQUE, 75016 Paris Cédex (FR); Basset, Geneviève LF, 67200 Oberhausbergen (FR)
(72) Inventeur: BASSET, Paul DI, deceased (FR); DIVE, Vincent, F-94160 St Mande (FR); CUNIASSE, Philippe, F-75020 Paris (FR); RIO, Marie-Christine, F-67400 Hillkirch (FR); YOTAKIS, Athanasios, 15342 Ag Paraskevi (GR); BEAU, Fabrice, F-91300 Massy (FR); VASSILIOU, Stamania, 16233 Athenes (GR)
(74) Mandataire: Poulin, Gérard
(86) Numéro de dépôt international: FR0000093
(87) Numéro de publication internationale: WO00043404

(56) Documents cités:
- WO-A-93/14112
- WO-A-98/03516
- FR-A- 2 676 059
- GOULET J L ET AL: "Inhibition of stromelysin-1 (MMP-3) by peptidyl phosphinic acids" BIOORG. MED. CHEM. LETT. (BMCLE8,0960894X);1994; VOL.4 (10); PP.1221-4, XP002117546 Merck Res.;Dep. Med. Chem. Res.; Rahway; 07064-0900; NJ; USA (US) cité dans la demande
- VASSILIOU S. ET AL.: "Phosphinic pseudo- tripeptides as potent inhibitors of matrix metalloproteinases: a structure-activity study" JOURNAL OF MEDICINAL CHEMISTRY., vol. 42, no. 14, - 15 juillet 1999 (1999-07-15) pages 2610-2620, XP002135494 AMERICAN CHEMICAL SOCIETY. WASHINGTON., US ISSN: 0022-2623

## Description

### Domaine technique

La présente invention a pour objet des pseudo-peptides phosphiniques, utilisables en particulier comme inhibiteurs très puissants et spécifiques des métalloprotéases à zinc matricielles (MMP), en particulier les MMP-11, MMP-2, MMP-9 et MMP-8. En revanche, ces pseudo-peptides apparaissent très peu actifs vis à vis des MMP-1 et MMP-7. Ainsi, ces inhibiteurs offrent la possibilité de ne bloquer in vivo qu'une sous-famille de MMP, et pourraient donc à cet égard s'avérer être moins toxiques que des inhibiteurs de MMP à très large spectre d'activité.

Ces pseudo-peptides peuvent avoir des applications dans le traitement de maladies caractérisées par la sur-expression des protéases matricielles, telles que la dégénérescence des tissus conjonctifs et articulaires, les arthrites rhumatoïdes, les ostéo-arthrites, l'anévrisme aortique et le cancer.

Sur la base d'études réalisées in vivo chez l'animal, ces inhibiteurs phosphiniques apparaissent pouvoir être utilisés dans des compositions pharmaceutiques pour bloquer la croissance de tumeurs primaires ou secondaires.

### Etat de la technique antérieure

Les métalloprotéases à zinc matricielles (MMP) représentent une famille d'enzymes capables collectivement de cliver l'ensemble des protéines de la matrice extracellulaire. Du fait de leur rôle sur les protéines de la matrice extracellulaire, ces enzymes jouent un rôle très important lors du développement et au cours de différents processus de remodelage tissulaire, tels que l'involution de la glande mammaire, la cicatrisation et l'extravasion de cellules spécialisées de la réponse immunitaire.

On connaît aujourd'hui chez l'homme une quinzaine d'enzymes appartenant à la famille des MMP :

| Matrixines | |
|---|---|
| MMP-1 | Collagénase interstitielle |
| MMP-8 | Collagénase de neutrophile |
| MMP-13 | Collagénase 3 |
| MMP-18 | Collagénase 4 |
| MMP-3 | Stromélysine 1 |
| MMP-10 | Stromélysine-2 |
| MMP-11 | Stromélysine-3 |
| MMP-2 | Gelatinase-A |
| MMP-9 | Gélatinase-B |
| MMP-12 | Métalloélastase |
| MMP-14 | MT-1 MMP |
| MMP-15 | MT-2 MMP |
| MMP-16 | MT-3 MMP |
| MMP-17 | MT-4 MMP |
| MMP-7 | Matrilysine |

Les collagénases apparaissent comme les seules MMP à être capables de cliver le collagène sous forme fibrillaire. Les gélatinases A et B se caractérisent par leur capacité à cliver du collagène de type IV, très abondant au niveau des membranes basales et du collagène sous forme dénaturée. Les stromélysines 1 et 2 seraient responsables de la dégradation d'autres protéines de la matrice extracellulaire, comme la fibronectine et différentes protéoglicanes. Les MT-MMP seraient surtout impliquées dans l'activation de la gélatinase A, et du fait de leur localisation membranaire, ces matrixines joueraient le rôle de récepteur membranaire de la gélatinase A. Il est à noter que le substrat physiologique de la stromélysine-3 n'est toujours pas connu. Néanmoins, plusieurs travaux sur cette protéase, qui a été initialement caractérisée dans des tumeurs du sein, suggèrent que la stromélysine-3 est un facteur important du développement et de la survie des tumeurs.

Les MMP apparaissent sur-exprimées dans différentes pathologies chez l'homme, notamment le cancer. Dans cette pathologie, le rôle des MMP a longtemps été associé à l'invasion des cellules tumorales et à leur capacité à franchir différentes barrières pour former des tumeurs secondaires. Plus récemment, différentes études ont établi que ces protéases jouent certainement un rôle plus fondamental dans la cancérogénèse, en participant notamment à la croissance des tumeurs primaires. Parmi différentes hypothèses pour expliquer cette fonction des MMP, les plus étudiées concernent leur rôle dans :
- leur capacité à pouvoir, par protéolyse de protéines de la matrice extracellualire, libérer de cette matrice des facteurs de croissance indispensables au développement et à la survie des tumeurs, et
- l'angiogénèse, nécessaire à la croissance des tumeurs.

L'implication apparente des MMP dans la croissance des tumeurs a conduit de nombreuses équipes dans le monde à s'intéresser au rôle de composés capables d'inhiber ces enzymes.

Des programmes de synthèse sur les inhibiteurs de MMP ont été initiés depuis de nombreuses années. A cette époque, les applications sur les inhibiteurs de MMP visaient surtout les maladies inflammatoires des tissus conjonctifs. Ce n'est que plus récemment que de multiples programmes sur l'application des inhibiteurs de MMP en cancérologie se sont développés, comme il est décrit par Brown, Médical Oncology, 1997, 14, 1-10, [1]. En effet, comme rappelé ci-dessus, les travaux démontrant que les MMP doivent être considérées comme des cibles privilégiées pour développer de nouveaux agents anti-cancéreux sont relativement récents. A cet égard, on peut noter que pour l'année 1997, 67 brevets concernant la synthèse d'inhibiteurs de MMP ont été déposés dans le monde, dont la majorité concerne des applications en cancérologie, comme il est décrit par Beckett et al, 1998, Exp. Opin. Ther. Patents, 8, p.259-289 [2]. Dans la plupart de ces brevets, les composés synthétisés appartiennent à la famille des dérivés pseudo-peptidiques comportant une fonction hydroxamate. Quelques brevets concernent des composés pseudo-peptidiques possédant des fonctions carboxyl-alkyle ou thiol. Dans ces composés, les fonctions hydroxamate, thiol ou carboxyl-alkyle interagissent avec l'atome de zinc présent dans le site actif des MMP. Les composés les plus avancés pour leur activité anticancéreuse ont été développés par la firme British Biotechnology. Deux composés, le Batismatat BB94 et le Marimastat ont fait l'objet d'études cliniques en phases II et III chez l'homme. Depuis, il apparaît que d'autres firmes (Roche, Bayer, Agouron, Novartis) ont conduit des études cliniques en phases I et II sur des inhibiteurs de MMP en cancérologie.

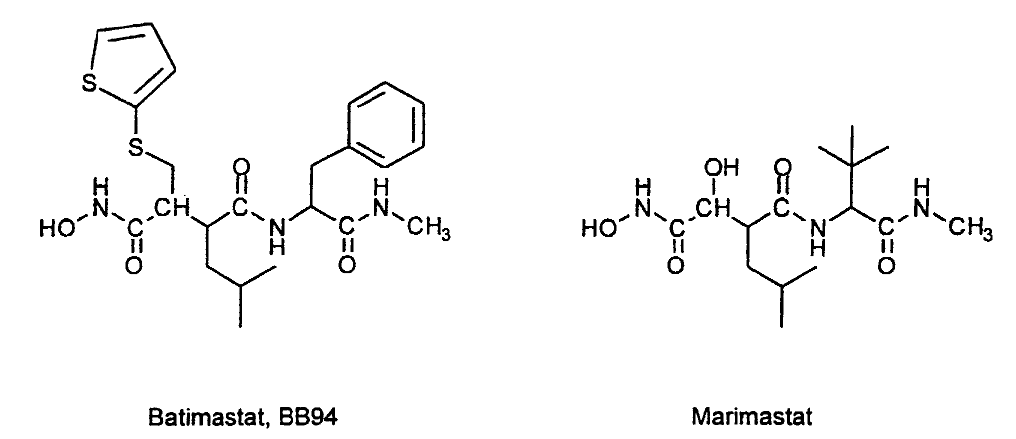

Ainsi, aucun des composés susceptibles d'inhiber les MMP connus par les références [1] et [2], n'est constitué par un pseudopeptide phosphinique.

Le document : Goulet et al, Bioorg. Med. Chem. Lett. 4, 1994, p. 1221-1224 [3], décrit toutefois des pseudo-peptides phosphiniques utilisables comme inhibiteur sélectif de la stromélysine-1 (MMP-3), qui comportent la séquence finale :

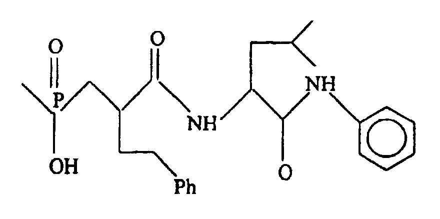

Caldwell et al, Bioorg. Med. Chem. Letter 6, 1996, p.323-328 [4], décrivent également un pseudo-peptide phosphinique qui est un inhibiteur sélectif de la stromélysine-1 (MMP-3), qui comporte la même séquence terminale que le pseudo-peptide de la référence [3].

Dans ces références [3] et [4], on recherche une activité vis-à-vis de la MMP-3, alors que dans l'invention, on recherche une activité sélective vis-à-vis des MMP-11, MMP-2, MMP-9 et MMP-8. On remarque d'autre part que dans la présente invention, R³ n'est pas seulement un résidu phényléthyle. Qui plus est, l'invention démontre que d'autres substituants dans cette position donnent des composés au pouvoir inhibiteur renforcé.

Les documents FR-A- 2 676 059 [5], FR-A-2 689 764 [6] et EP-A-0 725 075(7] illustrent des pseudo-peptides phosphiniques présentant une activité inhibitrice vis-à-vis des collagénases bactériennes et des endopeptidases à zinc 24.15 et 24.16.

Le document WO 98/03516 décrit des inhibiteurs de métalloprotéases matricielles à base de phosphinate répondant à la formule : 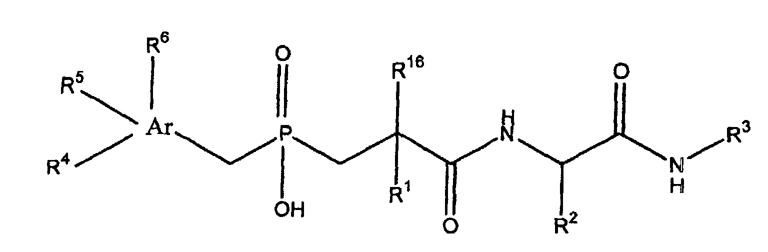 dans laquelle R¹, R², R³, R⁴, R⁵, R⁶ et R¹⁶ peuvent représenter de très nombreux groupements.

Le document WO 93/14112 décrit des dérivés peptidiques phosphiniques répondant à la formule : 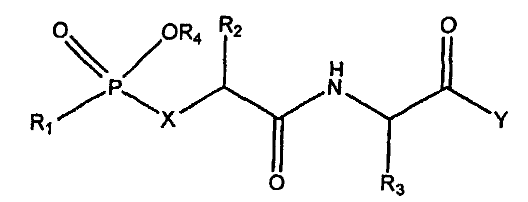 dans laquelle X, Y, R₁, R₂, R₃ et R₄ peuvent avoir de très nombreuses significations, qui sont utiles pour le traitement de maladies mettant en cause des métalloendoprotéinases matricielles.

La présente invention a précisément pour objet des nouveaux pseudo-peptides phosphiniques présentant une activité inhibitrice puissante et spécifique vis-à-vis des métallo-protéases à zinc matricielles MMP-11, MMP-2, MMP-9 et MMP-8.

### Exposé de l'invention

Selon l'invention, le pseudo-peptide phosphinique répond à la formule : 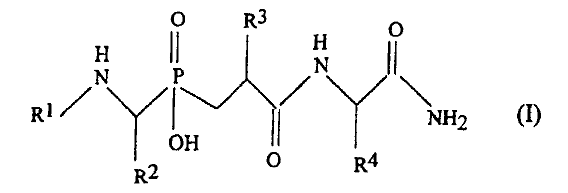 dans laquelle
- R¹ est un groupe bloquant une fonction aminé, ou un résidu d'aminoacide ou de peptide à fonction amino terminale bloquée tel que défini à la revendication 1,
- R² représente la chaîne latérale d'un acide aminé naturel ou non naturel, telle que définie à la revendication 1,
- R³ représente :
   1) la chaîne latérale d'un acide aminé naturel sauf Gly et Ala, non substituée ou substituée par un groupe aryle,
   2) un groupe aralkyle, ou
   3) un groupe alkyle d'au moins 3 atomes de carbone, et
- R⁴ représente une chaîne latérale d'acide aminé naturel ou non naturel telle que définie à la revendication 1, ou un groupe dinitrobenzyle.

Ce pseudo-peptide de formule (I) est un pseudo-tripeptide possédant un groupement chimique de type phosphinique qui a pour fonction de chélater l'atome de zinc des MMP. Dans ce pseudo-tripeptide, le choix des groupes R², R³ et R⁴ permet d'assurer l'interaction du tripeptide respectivement avec les sous-sites S1, S1' et S2' des MMP. Le groupe R¹ est supposé interagir à la jonction des sous-sites S3/S2.

Dans la définition donnée ci-dessus des pseudo-peptides de l'invention, les termes « aminoacide » utilisés pour R¹, R²_{,} R³ et R⁴ dans [5] se rapportent aux vingt α-aminoacides communément trouvés dans les protéines qui sont également connus sous le nom d'aminoacides standard et à leurs analogues. Les chaînes latérales de ces aminoacides comprennent des groupes alkyle linéaires et ramifiés, hydroxyalkyle, carboxyalkyle, aralkyle, aminoalkyle, carboxamide alkyle, mercapto alkyle, phénylalkyle, hydroxyphénylalkyle, guanidinoalkyle, imidazoylalkyle, indolylalkyle, et pyrrolidinyle.

A titre d'exemple d'acides aminés utilisables, on peut citer l'alanine, l'arginine, l'asparagine, l'acide aspartique, la cystéine, la glutamine, l'acide glutamique, la glycine, l'histidine, l'isoleucine, la leucine, la norleucine, la lysine, la méthionine, la phénylalanine, la proline, l'hydroxyproline, la sérine, la thréonine, le tryptophane, la tyrosine, la valine, la nitrophénylalanine, l'homo arginine, la thiazolidine et la déshydroproline.

Toutefois, et dans le cas de R³, l'acide aminé ne peut être Gly ou Ala car ceux-ci ne présentent pas une interaction suffisante avec le sous site S1' des MMP.

De préférence, l'acide aminé utilisé pour R³ est choisi parmi Phe, Leu ou bien des résidus Ser, Cys dont la chaîne latérale est substituée par un groupe arylalkyle.

Les groupes aryle susceptibles d'être utilisés sont ceux dérivés d'un noyau aromatique monocylique ou polycyclique, éventuellement substitué par des groupes alkyle ou alcoxy. A titre d'exemples de groupes aryle utilisables, on peut citer les groupes phényle, naphtyle, benzyle et alcoxybenzyle tel que p-méthoxybenzyle.

R³ peut encore représenter un groupe aralkyle. Dans ce groupe aralkyle, le groupe aryle peut être l'un de ceux mentionnés ci-dessus. La partie alkyle du groupe aralkyle peut être une chaîne linéaire ou ramifiée de 1 à 6 atomes de carbone.

A titre d'exemple de groupes aralkyle utilisables, on peut citer les groupes de formules : 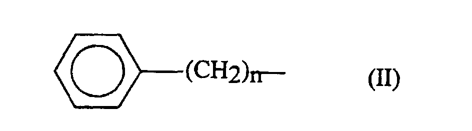 avec n étant un nombre entier de 1 à 4, et 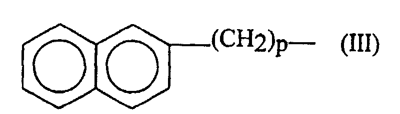 avec p étant égal à 1 ou 2.

Les groupes alkyle utilisables pour R³ ont au moins 3 atomes de carbone. Ils peuvent être linéairs ou ramifiés et ont de préférence au plus 7 atomes de carbone. A titre d'exemple de groupes alkyle utilisables, on peut citer les groupes CH₃―(CH₂)₆― et (CH₃)₂― CH―CH₂―.

De préférence, R³ représente un groupe répondant à l'une des formules suivantes :

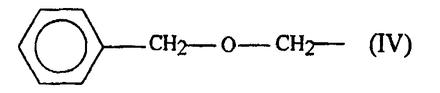

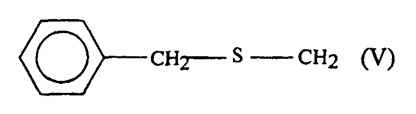

(CH₃)₂-CH-CH₂- (VI)

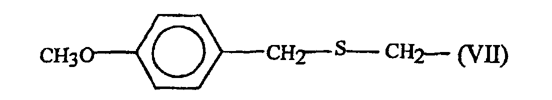

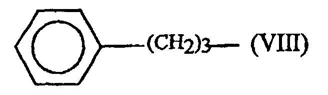

Selon l'invention, R² est choisi de façon à interagir avec le sous-site S1 des MMP. De bons résultats sont obtenus lorsque R² représente le groupe méthyle ou benzyle ; de préférence R² est le groupe benzyle, ce qui correspond à la chaîne latérale de Phe.

Selon l'invention, R⁴ est choisi de façon à interagir avec le sous-site S2' des MMP. De bons résultats sont obtenus lorsque R⁴ représente la chaîne latérale de Trp ou un groupe dinitrobenzyle (Dpa).

Dans le pseudo-peptide de l'invention, les chaînes latérales R², R³ et R⁴ des acides aminés peuvent être sous forme L ou D. Aussi, le pseudo-peptide peut être constitué par un seul isomère ou par un mélange de 4-diastéroisomères provenant de la présence de deux centre asymétriques au niveau des carbone α portant les résidus R² et R³. Bien que toute configuration des acides aminés puisse convenir, il est préférable que le carbone asymétrique de l'acide aminé : 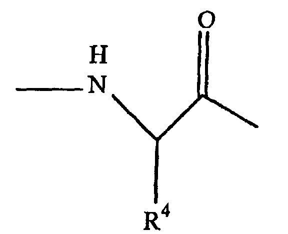 soit sous forme L.

En revanche, trois sur quatre diastéroisomères correspondant aux différentes configurations de R² et R³ ont des activités pratiquement équivalentes comme inhibiteurs des MMP.

Dans les pseudo-peptides de l'invention, R¹ peut représenter divers groupements dont la nature influence l'affinité du pseudo-peptide vis-à-vis des différentes MMP.

R¹ représente un « groupe bloquant une fonction amine ». Ces termes incluent les groupes t-butoxy-carbonyle, benzyloxycarbonyle, cinnamoyle, pivalolyle et N-(I-fluorényl-méthoxycarbonyle) Fmoc.

R¹ représente des groupes bloquants choisis parmi les groupes acétyle, benzyloxyacétyle, phénylaminoacétyle, (m-chlorophényl)aminoacétyle, (2-hydroxy-5-chloro-phényl) amino acétyle, indolyl-2-carbonyle, 4,6-dichloro-indolyl-2-carbonyle, quinolyl-2-carbonyle et 1-oxa-2,4-dichloro-7-naphtalène carbonyle,
ou encore un résidu d'aminoacide ou de peptide dont la fonction aminé terminale est bloquée par un groupe approprié. A titre d'exemple de tels résidus, on peut citer les groupes Z-Ala et Z-Leu dans lesquels Z représente le groupe benzyloxycarbonyle.

Les pseudo-peptides de l'inventin peuvent être préparés par des procédés classique à partir des blocs phosphiniques de formule : 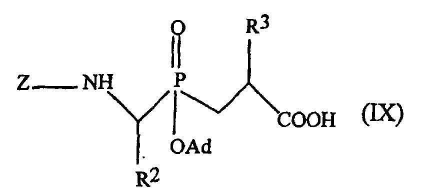 dans laquelle Z représente le groupe benzyloxycarbonyle et Ad le groupe adamantyle, et de l'aminoacide correspondant à R⁴ par synthèse chimique en phase solide selon les procédés décrits par Yotakis et al, J. Org. Chem., 1996, 61, page 6601-6605 [8] et Jiracek et al, J. Biol. Chem., 1995, 270, p. 21701-21706 [9] et J. Biol. Chem., 1996, 271, p. 19606-19611 [10].

Les blocs phosphiniques de départ peuvent être obtenus par addition de Michael d'un acide phosphinique porteur du groupe R² 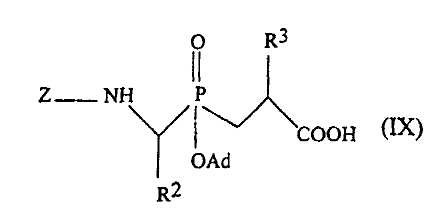 sur un acrylate fournissant le groupe R³ 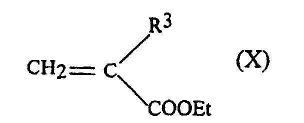 avec Et représentant le groupe éthyle.

Les acrylates founissant le groupe R³ peuvent être synthétisés par différentes voies, comme on le verra ci-après.

Les pseudo-peptide de l'invention peuvent être utilisés pour le traitement de maladies mettant en jeu une surexpression des métalloprotéases à zinc matricielles.

Aussi, l'invention a encore pour objet une composition pharmaceutique inhibant au moins une métalloprotéase à zinc matricielle, comprenant au moins un pseudo-peptide de formule (I) tel que défini ci-dessus.

De préférence, cette composition inhibe une métalloprotéase à zinc matricielle choisie parmi les MMP-2, MMP-8, MMP-9 et MMP-11.

Cette composition est destinée au traitement d'une maladie caractérisée par la surexpression des protéases matricielles, telles que le cancer.

L'invention concerne aussi l'utilisation d'un pseudo-peptide phosphinique de formule (I) tel que défini ci-dessus, pour la fabrication d'un médicament inhibant au moins une métalloprotéase à zinc matricielle, en particulier les MMP-2, MMP-8, MMP-9 et MMP-11.

D'autres caractéristiques et avantages de l'invention apparaîtront mieux à la lecture de la description qui suit en référence aux dessins annexés.

### Brève description des dessins

La figure 1 est un schéma de synthèse de pseudo-peptides conformes à l'invention.
La figure 2 est un diagramme illustrant l'efficacité anti-tumorale d'un composé de l'invention, qui donne le volume moyen de la tumeur (en mm³) en fonction du temps (en jours) après l'injection des cellules cancéreuses sur des souris témoins et des souris traitées par le pseudo-peptide de l'invention.

### Exposé détaillé des modes de réalisation

Les exemples 1 à 27 qui suivent, illustrent la fabrication de pseudo-peptides conformes à l'invention.

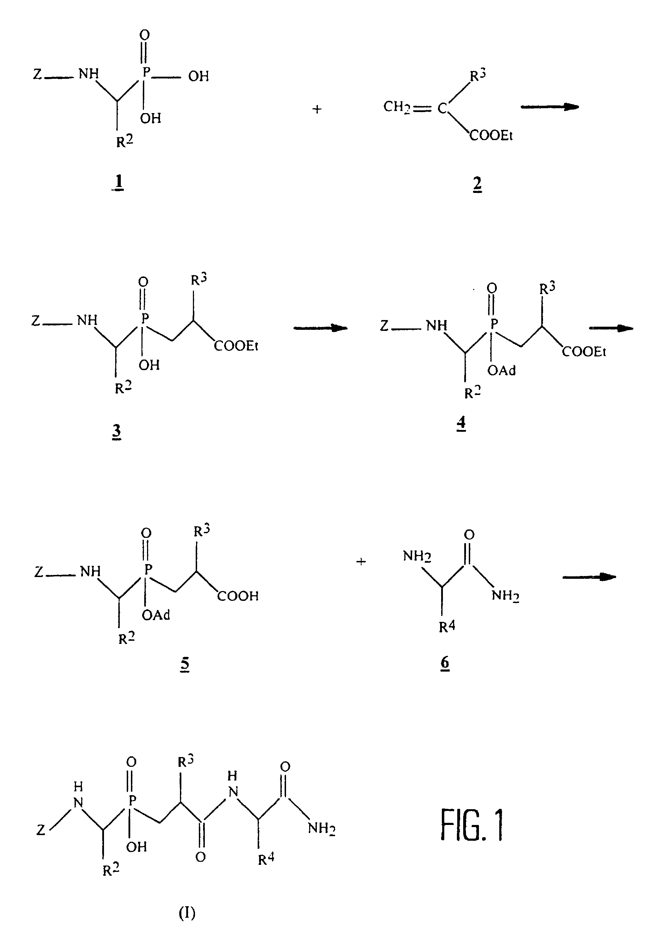

Sur la figure 1, on a représenté le schéma de synthèse des pseudo-peptides de l'invention, qui met en jeu une première réaction pour la fabrication de blocs phosphiniques **3** à partir d'un acide phosphinique **1** porteur du groupe R² et d'un acrylate **2** fournissant le groupe R³, par addition de Michael.

Après formation du bloc phosphinique **3**, on introduit un groupe adamantyle sur la fonction hydroxyphosphinyle du bloc **4**, puis on élimine la fonction ester C-terminale **5**, et on effectue une synthèse en phase solide du pseudo-peptide par addition de l'acide aminé souhaité **6** sur la fonction acide C-terminale.

Sur cette figure Z représente le groupe benzyloxycarbonyle.

### Exemples 1 à 5

Ces exemples illustrent la préparation d'acrylates **2** comportant un groupe R³ avec CH₂ terminal par le procédé suivant :

### Voie A :

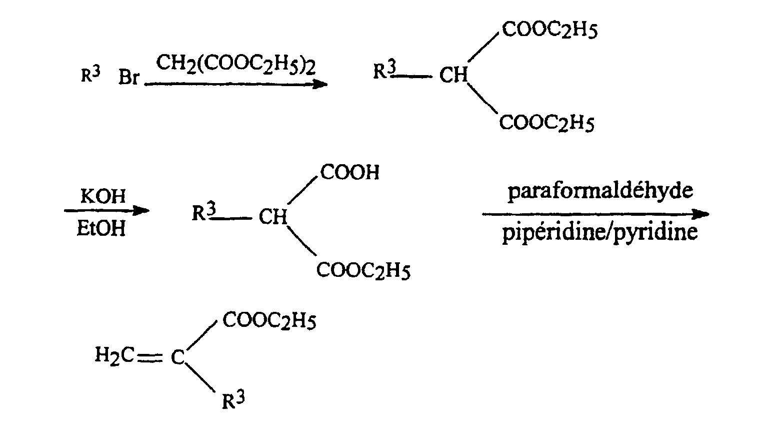

Cette synthèse correspond au procédé décrit par Eistetter et al dans J. Med. Chem., 25, p109-113, 1982 [11].

On décrit ci-après cette synthèse dans le cas où R³ représente les groupes illustrés dans le tableau 1.

A une solution d'éthoxyde de sodium (10 mmol de sodium dans 11 ml d'éthanol absolu), on ajoute sur une période de 10 minutes, 10 mmol de malonate de diéthyle. On soumet la solution à une agitation à 50°C, pendant 1 heure, puis on y ajoute goutte à goutte 10 mmol du bromure requis. On soumet le mélange à une agitation pendant 6 heures à 50°C, puis on élimine l'éthanol sous vide et on ajoute de l'éther diéthylique. On lave cette solution avec de l'eau, de la saumure et on sèche sur Na₂SO₄, puis on concentre pour obtenir un résidu huileux, qui après distillation donne le diester de formule : 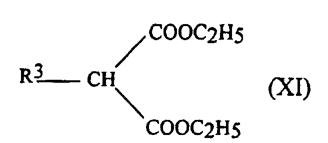 avec des rendements de 40 à 65 %.

A une solution de 10 mmol de diester dans 8 ml d'éthanol, on ajoute une solution de KOH (10 mmol) dans 8 ml d'éthanol, on agite le mélange pendant 16 heures. Après évaporation du solvant organique, on traite le résidu avec de l'eau et on l'extrait avec de l'éther diéthylique.

On acidifie la phase aqueuse avec HCl6N et on l'extrait 2 fois avec de l'éther diéthylique. On sèche les phases organiques sur Na₂SO₄ et on les évapore pour obtenir les monoesters de formule : 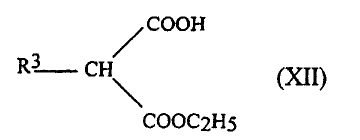 avec des rendements de 65 à 90 %.

A une solution du monoester dans 0,8 ml de pyridine et 0,05 ml de pipéridine, on ajoute 6 mmol de paraformaldéhyde, puis on soumet le mélange à une agitation et à un chauffage à 50-55°C pendant 3 heures. Après addition d'éther diéthylique, on lave la phase organique avec de l'eau et HCl3N, puis on sèche sur Na₂SO₄, et on concentre pour obtenir les composés **2a** à **2c** du tableau avec les rendements indiqués dans ce tableau.

Les points de fusion des acrylates **2a** à **2e** obtenus sont également donnés dans le tableau 1.

### Exemple 6

Dans cet exemple où R³ représente CH₂R'³ on réalise la synthèse de l'acrylate selon la voie de synthèse suivante :

### Voie B :

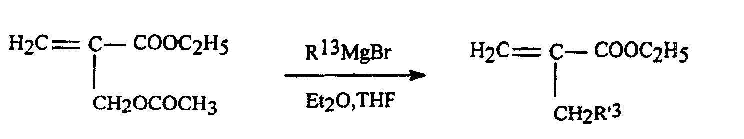

Dans cet exemple, on utilise cette voie de synthèse pour préparer l'acrylate **2** dans lequel R³ représente le groupe CH₃(CH₂)₆, soit R'³ = CH₃―(CH₂)₅. Sous atmosphère d'argon, on ajoute goutte à goutte une solution de bromure d'hexyle CH₃ (CH₂)₅ Br (1, 65 g, 10 mmol) dans 15 ml d'éther diéthylique absolu à un flacon contenant 0,22 g (11 mmol) de magnésium et de l'iode I₂ (catalytique) sur une période de 90 minutes.

On porte le mélange réactionnel au reflux pendant 1 heure. Après addition de 0,18 mmol de CuI, on fait décroître la température du mélange à -78°C.

On ajoute alors lentement une solution de 1,15 g (6,7 mmol) du composé : 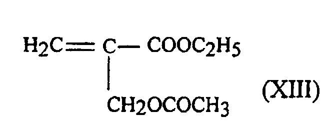 dans 10 ml de Et₂O : THF. On agite le mélange pendant 30 minutes à la température ambiante. Après traitement du mélange avec HCl 0,5N, NaHCO₃ 5 % et de l'eau, on sèche la couche organique sur Na₂SO₄. On élimine le solvant sous vide et on purifie le résidu obtenu par chromatographie sur colonne en utilisant comme éluant un mélange d'éther de pétrole 40 à 60°C/éther (13 : 1). On obtient ainsi le composé **2f** avec un rendement de 40 %. Les caractéristiques du composé **2f** sont données dans le tableau 1.

### Exemple 7

Dans cet exemple, on prépare un acrylate 2 avec R₃ = R' ³―CH₂―, en utilisant le procédé décrit par Baldwin et al J. Chem. Soc. Chem. Comm. 1986, p 1339-1340, [12].

Ceci correspond au schéma réactionnel suivant :

### Voie C :

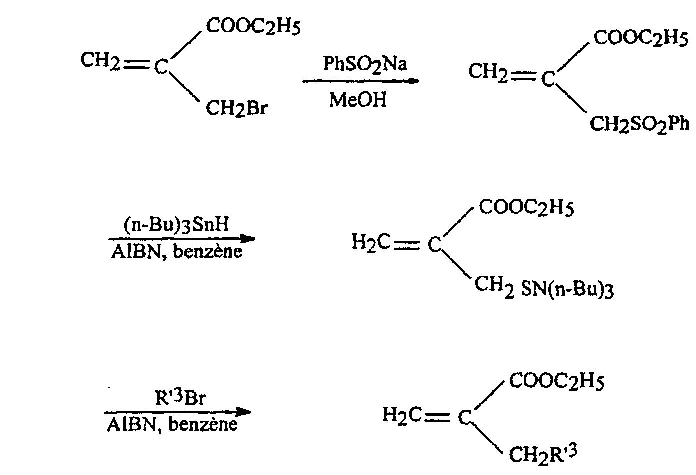

Ce procédé est utilisé pour préparer l'acrylate **2g** dans lequel R³ représente le groupe de formule :

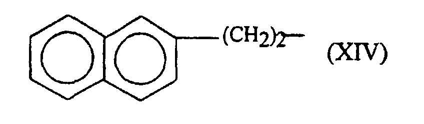

On porte au reflux pendant 12 heures un mélange de 1,9 g (10 mmol) de bromométhyl acrylate d'éthyle et de 3,3 g (20 mmol) du sel de sodium de l'acide benzène sulfinique dans 40 ml de méthanol. On élimine le solvant et on ajoute de l'éther diéthylique. On lave la solution avec de l'eau, de la saumure, on sèche sur Na₂SO₄ et on concentre pour obtenir 2 grammes d'acrylate sulfinique 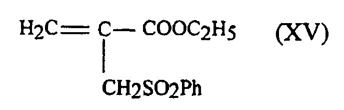 sous forme d'huile, avec un rendement de 80 %.

A un mélange de 2 g (8 mmol) d'acrylate sulfinique dans 40 ml de benzène sec, on ajoute 4,7 g (16,4 mmol) d'hydrure de tri-n-butyl étain et 0,15 g (0,96 mmol) de 2,2'-azobisisobutyronitrile (AIBN). On porte le mélange au reflux pendant 1,5 heure, puis on ajoute de l'eau et on sèche la couche organique extraite sur Na₂SO₄ et on la concentre. On purifie le produit brut par chromatographie sur colonne, en utilisant comme éluant l'éther de pétrole (40 à 60°C)/éther (9 : 1). On obtient 2,9 g d'alkylstannane 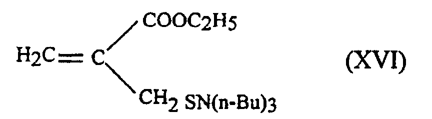 avec un rendement de 90 %.

On dissout dans 10 ml de benzène sec 1,47 g (3,6 mmol) d'alkylstannane et 0,39 g (1,8 mmol) de 2-bromoéthyl naphtalène. Après addition de 0,064 g (0,39 mmol) de AIBN, on porte le mélange réactionnel au reflux pendant 2 heures. Après addition d'eau, on sépare la phase organique, on la lave avec de l'eau, on la sèche sur Na₂SO₄ et on la concentre à siccité. On obtient ainsi 0,06 g de l'acrylate **2g** donné dans le tableau 1, avec un rendement de 13 %, après purification du résidu sur colonne de chromatographie en utilisant l'éther de pétrole (40 à 60°C)/éther (9 : 1) comme éluant.

### Exemples 8 et 9

Dans ces exemples, on prépare un acrylate **2** dans lequel R³ comporte un atome de soufre ou un atome d'oxygène, en suivant le schéma de synthèse suivant :

### Voie D :

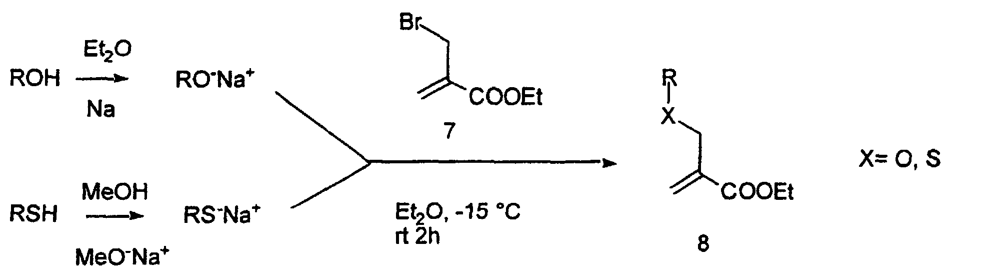

Dans ce schéma, R³ correspond à RX―CH₂ avec X = S ou O.

On utilise ce procédé pour préparer les acrylates **2h** et **2i** dans lesquels R³ représente respectivement le groupe paraméthoxybenzylthiométhyle et le groupe benzylthiométhyle.

On ajoute goutte à goutte une solution de 10 mmol du thiol approprié R-SH dans 15 ml de méthanol à une solution agitée refroidie dans un bain de glace de 9 mmol de sodium dans 20 ml de méthanol sur une période de 30 minutes. Après addition du thiol, on concentre le mélange à sec et on ajoute de l'éther diéthylique. On refroidit le sel qui précipite dans un bain de glace. On filtre le produit, on le lave avec Et₂O froid et on le sèche sur P₂O₅ pour obtenir le sel de sodium avec un rendement de 85 à 95 %. A un mélange de 10 mmol de sel de sodium en suspension dans 40 ml de Et₂O sec et refroidi dans un bain de glace, on ajoute goutte à goutte, sur une période de 45 minutes, une solution de bromométhacrylate d'éthyle (9 mmol) dans 20 ml d'éther diéthylique. On agite la solution pendant 30 minutes à 0°C et pendant 1 à 2 heures à la température ambiante. On dilue le mélange réactionnel avec 20 ml d'eau et on lave la couche organique avec de l'eau, puis on la sèche sur Na₂SO₄ et on évapore. On purifie les composés **2h** et **2i** ainsi obtenus par chromatographie sur colonne, en utilisant comme éluant de l'éther de pétrole (40-60°C)/Et₂O (8 : 2).

Les rendements sont donnés dans le tableau 2.

### Exemple 10

Dans cet exemple, on prépare l'acrylate **2j** dans lequel R³ correspond à ROCH₂ et représente le groupe de formule C₆H₄―CH₂―O―CH₂, en suivant le schéma réactionnel illustré ci-dessus, avec R OH.

Dans un flacon bien sec, on ajoute des petits morceaux de sodium (10 mmol) à 20 ml d'éther diéthylique sec. On ajoute à ce mélange réactionnel, une solution de 10 mmol d'alcool benzylique dans 10 ml d'éther diéthylique sur une période de 2 heures, sous un reflux modéré. On porte le mélange au reflux pendant 6 heures supplémentaires. On refroidit dans un bain de glace le précipité blanc obtenu, puis on filtre, on lave avec de l'éther diéthylique sec et froid et on sèche sur P₂O₅ pour obtenir le sel de sodium avec un rendement de 85 à 95 %.

La réaction de ce sel avec le bromométhacrylate d'éthyle et la purification du composé obtenu sont effectués comme il est décrit dans l'exemple 8.

L'acrylate **2j** obtenu est répertorié dans le tableau 2

### Exemples 11 à 21

Dans ces exemples, on prépare des blocs phosphiniques **3** avec R² et R³ donnés dans le tableau 3, en utilisant la procédure décrite par Yiotakis et al, J. Org. Chem. 61, 1996, 6601-6605 [8].

Cette procédure comporte dans un premier temps la réaction de Michael décrite sur la figure 1. L'acide phosphinique 1 de départ (figure 1) a été préparé selon le procédé décrit par Baylis et al, J. Chem. Soc. Perkin Trans I, 1984, p. 2845-2853 [13].

Les acrylates **2** ont été préparés dans les exemples 1 à 10. Pour la préparation des blocs phosphiniques, on opère de la façon suivante.

On chauffe à 110°C, pendant 1 heure, sous atmosphère d'azote, une suspension de 1 mmol d'acide N-benzoyioxycarbonylphosphinique **1** et de 5 mmol d'hexaméthyldisilazane.

On ajoute ensuite goutte à goutte 1,3 mmol de l'acrylate **2** approprié, sur une durée de 15 minutes. On soumet le mélange réactionnel à une agitation pendant 3 heures supplémentaires à 110°C. On laisse refroidir le mélange à 70°C et on ajoute goutte à goutte 3 ml d'éthanol. Après refroidissement à la température ambiante, on concentre le mélange réactionnel. On purifie le résidu par chromatographie sur colonne en utilisant comme éluant un mélange chloroforme/méthanol/acide acétique (7 : 0,5 : 0,5). On obtient ainsi les blocs phosphiniques **3a** à **3k** avec les rendements donnés dans le tableau 3.

Dans ce tableau, on a indiqué dans la colonne R³ quel acrylate **2** était utilisé pour la synthèse du bloc phosphinique. Le tableau 3 donne également le Rf des blocs obtenus.

### Exemples 22 et 23.

Dans ces exemples, on synthétise les blocs phosphiniques **3l** et **3m** répondant aux formules données dans le tableau 3 de la façon suivante.

On ajoute 2,1 mmol de diisopropylamine et 2,1 mmol de chlorure de triméthylsilyle à une solution refroidie à la glace de 1 mmol d'acide phosphinique **1** dans 2 ml de chloroforme.

On soumet le mélange à une agitation à la température ambiante pendant 3 heures. Après refroidissement à 0°C, on ajoute 1,4 mmol de l'acrylate d'éthyle **2** approprié (**2e** ou **2g**), puis on agite le mélange réactionnel à la température ambiante pendant 16 heures. Après avoir ajouté goutte à goutte de l'alcool éthylique, on élimine les solvants sous vide. On obtient les composés **3l** ou **3m** du tableau 3 après une chromatographie sur colonne comme dans le cas des exemples 11 à 21.

Les rendements et les Rf des blocs obtenus sont donnés dans le tableau 3.

Les blocs **3b**, **3d** et **3e** à **3k** ont de plus été caractérisés par RMN du proton, du carbone 13 et du phosphore. Les résultats obtenus figurent en annexe.

### Exemple 24

Dans cet exemple, on prépare les composés **4a** à **4m** de la figure 1 à partir des composés **3a** à **3m** en suivant le mode opératoire suivant.

On dissout dans 10 ml de chloroforme 1 mmol du composé **3a** à **3m** et 1,2 mmol de bromure de 1-adamantyle. On porte le mélange réactionnel au reflux. On ajoute ensuite 2 mmol d'oxyde d'argent en cinq parties égales, sur une période de 50 minutes. On porte la solution au reflux pendant 30 minutes supplémentaires. Après avoir éliminé les solvants, on traite le résidu avec de l'éther diéthylique et on filtre sur Célite. On concentre les filtrats. On purifie le résidu par chromatographie sur colonne en utilisant comme éluant le mélange chloroforme/isopropanol (9,8 : 0,2). On obtient ainsi les composés **4a** à **4m** avec les rendements indiqués dans le tableau 4, les Rf de ces composés sont également indiqués dans le tableau 4.

### Exemple 25

Dans cet exemple, on suit le mode opératoire décrit sur la figure 1 pour transformer les composés **4a** à **4m** en composé **5a** à **5m**. Dans ce but, on opère de la façon suivante.

On ajoute goutte à goutte 1 ml de NaOH 4N à une solution agitée de 1 mmol du composé **4a** à **4m** dans 5,5 ml de méthanol. On agite le mélange réactionnel pendant 18 heures, puis on élimine le solvant. On dilue le résidu avec de l'eau, puis on l'acidifie avec HCl 0,5 N dans un bain de glace, on sèche sur Na₂SO₄ et on concentre pour obtenir les composés **5a** à **5m** sous forme de solide blanc, avec les rendements donnés dans le tableau 5. Les Rf de ces composés sont également donnés dans le tableau 5.

### Exemple 26

Dans cet exemple, on prépare des pseudo-peptides phosphiniques dont les formules sont données dans les tableaux 5 à 7, à partir des blocs phosphiniques du tableau 4, par une synthèse en phase solide par chimie Fmoc (fluorénylméthoxycarbonyle), selon la procédure décrite par Yotakis et al, 1996, J. Org. Chem. 61, pages 6601-6605 [8] et Jiracek et al dans J. Biol. Chem., 1995, 270, pages 21701-21706 [9] et dans J. Biol. Chem., 1996, 271, pages 19606-19611 [10].

Le composé 12 du tableau 6 ne fait pas partie de l'invention.

On réalise les couplages par la stratégie in situ en utilisant le 2-(1H-benzotriazol-1-yl-1,1,3,3-tétraméthyluronium-hexafluorophosphate(HBTU)/diisopropyléthylamine. Les conditions de couplage sont les suivantes: trois équivalents de dérivé de Fmoc amino acide et 4 équivalents de diisopropyléthylamine dans le diméthylformamide sont ajoutés à la résine et laissés réagir pendant 30 min. Pour le couplage des blocs phosphiniques : 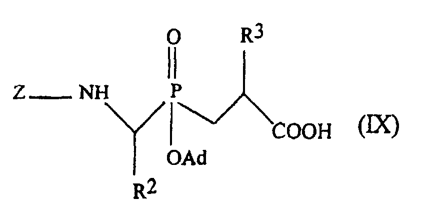 on utilise 1,5 équivalent de bloc.

Les conditions de clivages du groupe Fmoc sont 50 % de pipéridine dans le diméthylformamide pendant 30 minutes. Le groupe Fmoc est le (fluorémylméthoxy) carbonyle.

### Exemple 27

Dans cet exemple, on réalise la synthèse des pseudo-peptides figurant dans le tableau 8 qui comportent des R¹ de nature différente. Les conditions d'introduction du groupe R¹ sont décrites ci-dessous, selon la nature de ce groupe.

Dans le cas où R¹ est un acide comportant un groupe indole ou quinoline (composés 30, 31, 32, 33 du tableau 8), le couplage de R¹ sur le peptide de formule générique 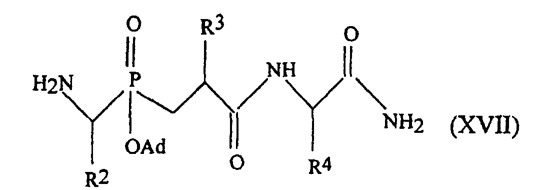 encore couplé à la résine, a été réalisé dans les conditions suivantes: 3 équivalents de l'acide dilué dans petit volume de N-méthyl-pyrolidone, 3 équivalents de HBTU (0,4 M), 10 équivalents de diisopropyléthylamine (1,2 M), avec un temps de couplage de 45 min. L'incorporation du groupe R¹ est suivie par un test de Kaiser. Lorsque nécessaire, cette opération a été répétée plusieurs fois, jusqu'à substitution complète de la fonction aminé.

L'incorporation du groupe R¹= Ph-CH₂-O-CH₂-CO- (composé 26) a été réalisée à partir du dérivé chloré correspondant avec les conditions suivantes: le dérivé chloré (25 équivalents, 0.5 M/dichlorométhane) et la diisopropyléthylamine (25 équivalents, 0,5 M/dichlorométhane) sont ajoutés sur la résine. La réaction d'acylation nécessite 1/2 h.

Pour la synthèse des composés 27, 28 et 29, le peptide 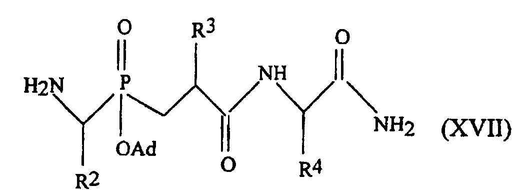 encore couplé à la résine, a été acylé tout d'abord avec le dérivé Br-CH₂-CO-Br dans les conditions suivantes: le dérivé bromé (25 équivalents, 0,5 M/dichlorométhane) et la diisopropyléthylamine (25 équivalents, 0,5 M/dichlorométhane) sont ajoutés sur la résine, pour un temps de réaction de 1/2 h. La deuxième étape correspond à l'alkylation des dérivés d'aniline correspondants. Pour ce faire, le dérivé aniline (50 équivalents/DMSO) est ajouté à la résine, pour un temps de couplage de 2,5 h.

Le clivage des peptides de la résine, ainsi que d'hydrolyse des groupements protecteurs ont été réalisés à l'aide d'une solution d'acide trifluoroacétique contenant 2,5 % d'eau, 2,5 % de thioanisol, 1,25% de thiophenol, 1,25 % d'éthanedithiol et 1,25% de triisopropylsilane.

Tous les peptides synthétisés dans les exemples 26 et 27 ont été purifiés par HPLC phase inverse à l'aide de gradients réalisés avec des solutions d'eau, d'acétonitrile, contenant 0,01 % d'acide trifluoroacétique. Dans la majorité des cas, on observe dans les chromatogrammes quatre pics, correspondant aux quatre formes de diastéréoisomères générés par le protocole de synthèse de ces composés phosphiniques. Tous les peptides phosphiniques ainsi purifiés ont été contrôlés par spectroscopie de masse.

### Exemple 28

Dans cet exemple, on examine les constantes d'affinité Ki des pseudo-peptides des tableaux 5 à 8 vis-à-vis des différentes métalloprotéases matricielles MMP.

Les MMP utilisées ont été produites sous-forme recombinante, dans un système d'expression E. coli, puis purifiées par différent types de chromatographies. A part la stromélysine-3 (MMP-11), les MMP produites correspondent aux séquences humaines. La Stromélysine-3 utilisée dans cette étude correspond à la forme murine.

L'activité de chaque MMP a été déterminée en utilisant deux substrats synthétiques fluorogéniques. La coupure de ces substrats, qui engendre un signal de fluorescence proportionnel à la quantité de substrat clivé, permet une détermination précise des paramètres cinétiques. Les valeurs des constantes Km des substrats, prises en compte pour déterminer la valeur des Ki, sont reportées dans le tableau qui suit.Les constantes d'affinité Ki ont été calculées à partir de l'équation de Horowitz et al, Proc. Natl. Acad. Sci. USA, 1987, 84, p. 6654-6658 [14], prenant en compte la dépendance du pourcentage d'inhibition mesuré expérimentalement selon la concentration de l'inhibiteur, en fonction de la concenration d'enzyme et de substrat.

Les expériences ont été réalisées dans un tampon Tris/HCl 50mM, pH 6,8, 10 mM CaCl₂, 25°C.

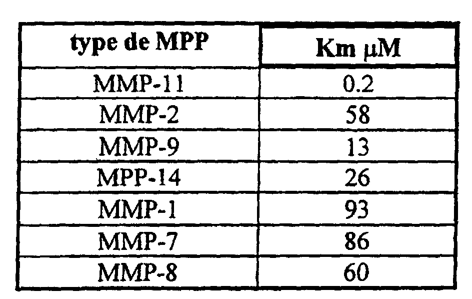

Les résultats obtenus sont donnés dans les tableaux 5 à 8.

La formule générique des inhibiteurs synthétisés ici indique qu'il s'agit de pseudo-tripeptides phosphiniques. D'après le mode de liaison supposé de ces composés avec le site actif des MMP, ces inhibiteurs doivent interagir respectivement avec les sous-sites S1, S1' et S2' de ces enzymes. Le groupement R1 figurant dans la plupart des inhibiteurs est supposé interagir à la jonction des sous-sites S3/S2.

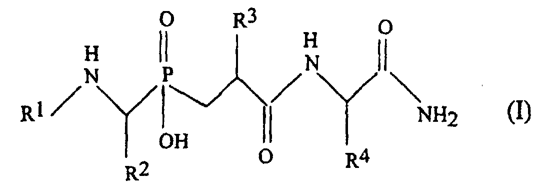

Dans le tableau 5, on a vérifié l'influence de la nature du résidu R³ sur l'efficacité du pseudo-peptide comme inhibiteur des MMP.

L'interaction des inhibiteurs avec le sous-site S1', impliquant le groupe R³ des composés, a fait l'objet d'une étude poussée, puisque ce sous-site est supposé contrôler en grande partie la sélectivité des interactions enzyme-inhibiteur.

L'analyse du tableau 5 démontre que la taille et la nature du résidu R³ joue un rôle critique pour l'affinité des composés: ainsi l'activité des inhibiteurs apparaît multipliée par un facteur 30 dans le cas de la MMP-8, lorsque le groupe R³ passe d'un résidu benzyle à phénylpropyle (composés **7** et **9**). On remarque que la gain d'affinité correspondant à cette substitution n'est pas constant pour les différentes MMP, suggérant que le sous-site S1' de chaque MMP peut être sensible à la nature du résidu R³. A cet égard, il est intéressant de constater que la MMP-11 est la seule MMP à préférer un résidu phénétyle, par rapport à un résidu phénylpropyle (composé **8** et **9**).

La comparaison des composés **9**, **10** et **11** qui ne diffèrent que par un seul atome ( C, O, S) au niveau du résidu R³, suggère l'existence d'une interaction spécifique entre l'atome de soufre en position y de R³ des inhibiteurs et le sous-site S1' des MMP. Quelle que soit la MMP, le composé **11** est toujours le plus puissant parmi ces trois inhibiteurs.

Dans le tableau 6, les inhibiteurs se caractérisent par la présence d'un résidu phényle en R², au lieu d'un résidu méthyle (tableau 5). La comparaison des tableaux 5 et 6 révèle que la présence d'un résidu benzyle permet d'augmenter globalement l'affinité des inhibiteurs. Dans le cas spécifique de la MMP-11, la substitution méthyle->benzyle conduit à une augmentation d'affinité beaucoup plus importante pour cette MMP, que pour les autres. Ce résultat indique la préférence du sous site S1 de la MMP-11 pour un résidu benzyle, par rapport à un résidu méthyle.

L'introduction d'une plus grande diversité en R³ dans cette série a permis de mieux cerner l'influence du résidu en R³. Cette série démontre que pour obtenir des inhibiteurs phosphiniques puissants vis à vis de certaines MMP, il faut introduire dans la position R³, une chaîne arylalkyle longue. Ainsi, le composé **18** est un exemple d'inhibiteur très puissant de la MMP-8, MMP-11, MMP-2 et MMP-9.

Par rapport à ces MMP, on remarque que les inhibiteurs reportés dans cette étude apparaissent moins puissants vis à vis de la MMP-14.

De plus, de façon générale, ces pseudo-peptides apparaissent très peu actifs vis-à-vis des MMP-1 et MMP-7.

On remarquera que le composé **14** possédant un résidu R3 phénétyle apparaît très puissant sur la MMP-11,mais qu'il est beaucoup moins actif sur les autres MMP.

Le tableau 7 illustre l'importance du résidu tryptophane au niveau de la position R⁴, ainsi que l'importance de la configuration du résidu tryptophane. Le composé **24** indique que le résidu tryptophane dans ces inhibiteurs peut être remplacé par un autre résidu aromatique Dpa (dinitrobenzyle) dans le cas de la MMP-11 et de la MMP-8

Le tableau 8 illustre l'effet de différentes modifications au niveau du groupe R¹ (Table IV). L'analyse des résultats indique que la nature de R¹ influence l'affinité des composés vis à vis des différentes MMP. Le composé **31** est un exemple d'inhibiteur très puissant vis-à-vis de la MMP-11, présentant une certaine sélectivité pour cette enzyme. Les composés **34** et **35** représentent des exemples d'inhibiteurs puissants dans lesquels R¹ correspond à un acide aminé naturel.

### Exemple 29

Dans cet exemple, on étudie l'influence de la configuration des deux positions R² et R³ sur l'efficacité des pseudo-peptides comme inhibiteur des MMP.

La voix de synthèse utilisée dans les exmples précédents pour préparer les dérivés phosphiniques de ce brevet aboutit à l'obtention de chaque inhibiteur sous forme d'un mélange de quatre diastéréoisomères, provenant de la présence de deux centres asymétriques au niveau des carbone alpha portant les résidus R² et R³. Afin d'évaluer l'influence de la configuration de ces deux positions, le composé **15** a été resynthétisé en partant de l'acide aminé phénylalanine phosphinique optiquement pur, de configuration R ou S. Chaque synthèse aboutit à un mélange de deux diastéréoisomères:
Z-(**S**)PheY(PO₂CH₂)(**S**)pPhe-Trp-NH₂ et Z-(**S**)PheY(PO₂CH₂)(**R**)pPhe-Trp-NH₂
   ou
Z-(**R**)PheY(PO₂CH₂)(**S**)pPhe-Trp-NH₂ et Z-(**R**)PheY(PO₂CH₂)(**R**)pPhe-Trp-NH₂
facilement séparables par HPLC en phase inverse. Le tableau 9 présente l'activité des quatre diastéréoisomères du composé **15** vis-à-vis des différentes MMP. Il est intéressant de remarquer que pour cette classe de composés phosphiniques, au moins trois diastéréisomères inhibent de façon presque équivalente les différentes MMP. Cette propriété, en dehors d'un moyen de contrôler le sélectivité des inhibiteurs, pourrait s'avérait être aussi très importante en terme de métabolisme et de pharmacocinétique, deux paramètres pouvant être sensibles à la stéréochimie des molécules.

Les résultats de la caractérisation par RMN du proton, du carbone 13 et du phosphore de la fraction RI du composé 15 sont donnés en annexe.

### Exemple 30

Dans cet exemple, on mesure l'efficacité anti-tumorale du composé **15** (fraction RI) du tableau 9 (RXPO3).

Le modèle de tumorigénèse utilisé pour tester les effets de cet inhibiteur RXPO3 *in vivo* consiste en une greffe sous-cutanée de cellules malignes murines C26 chez des souris syngéniques (possédant un fond génétique compatible avec celui des cellules injectées).

Les cellules C26 établies à partir d'un cancer colique de souris Balb c (Corbett et al., 1975, Cancer Res, 35:2434-2439 [15] sont cultivées jusqu'à subconfluence. Après trypsinisation, les cellules sont centrifugées à 1000g pendant 10 minutes. Le culot est lavé et remis en suspension dans du PBS 1X. Un volume de 200 ml, contenant 5 x 10⁴ cellules C26, est injecté en sous-cutané en 2 endroits du dos de souris agées de 8 à 9 semaines. L'inhibiteur est solubilisé dans du PBS 1X et l'administration de 150 mg d'inhibiteur dans un volume de 150ml se fait par voie intrapéritonéale. Le traitement commence le jour de l'injection des cellules C26 et se poursuit à raison d'une injection par jour pendant 25 jours. Les volumes tumoraux sont mesurés tous les jours.

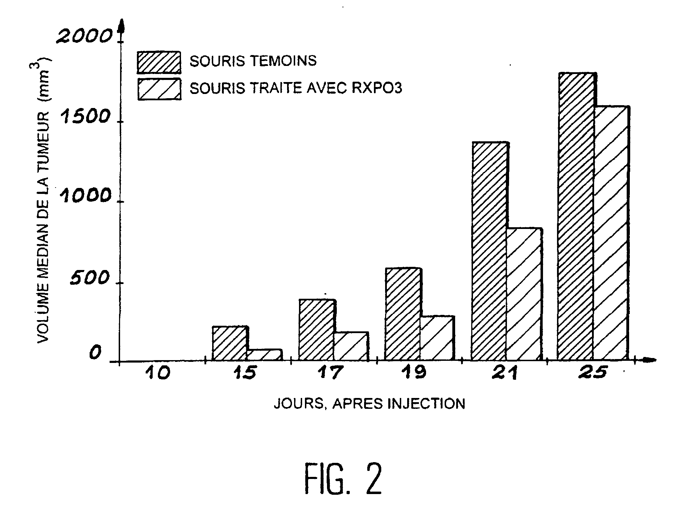

Trois expériences identiques et indépendantes de tumorigénèse ont été réalisées, selon le protocole précité. Les résultats obtenus étaient similaires. L'une d'entre-elles est rapportée ci-dessous.
L'expérience a concerné 12 animaux, 6 d'entre eux recevant un traitement (150 µg d'inhibiteur/souris/jour) et 6 servant d'animaux témoins (150 µl PBS 1X). La figure 2 montre les valeurs médianes des volumes tumoraux en fonction du temps écoulé depuis l'injection des cellules C26 (Jours 10 à 25).

On observe que les tumeurs commencent à apparaître au jour J10, et que leurs volumes moyens et médians sont toujours inférieurs chez les animaux traités par l'inhibiteur RXPO3. Cette différence de taille des tumeurs est de l'ordre de 50% du jour J15 au jour J20. Par la suite, l'efficacité de l'inhibiteur apparaît moindre. Ces résultats sont en accord avec les récentes observations faites sur un autre modèle de tumorigénèse mettant en oeuvre des animaux sauvages ou déficients pour l'expression de la stromélysine-3 (MMP-11) et montrant que cette métalloprotéase matricielle est impliquée dans les étapes initiales d'implantation et favorise le développement des tumeurs. D'après ce modèle, l'efficacité des inhibiteurs doit être maximale durant les premiers jours du développement de la tumeur.

### REFERENCES CITEES

[1] : Brown, Médical Oncology, 1997, 14, p. 1-10.
[2] : Beckett et al, 1998, Exp. Opin. Ther. Patents, 8, p. 259-289.
[3] : Goulet et al, Bioorg. Med. Chem. Lett. 4, 1994, p. 1221-1224.
[4] : Caldwell et al, Bioorg. Med. Chem. Letter 6, 1996, p.323-328.
[5] : FR-A- 2 676 059.
[6] : FR-A-2 689 764.
[7] : EP-A-0 725 075
[8] : Yotakis et al, J. Org. Chem., 1996, 61, p. 6601-6605.
[9] : Jiracek et al, J. Biol. Chem., 1995, 270, p. 21701-21706.
[10] : J. Biol. Chem., 1996, 271, p. 19606-19611.
[11] : Eistetter et al, J. Med. Chem., 25, p 109-113, 1982.
[12] : Baldwin et al, J. Chem. Soc. Chem. Comm. 1986, p 1339-1340.
[13] : Baylis et al, J. Chem. Soc. Perkin Trans I, 1984, p. 2845-2853.
[14] : Horowitz et al, Proc. Natl. Acad. Sci. USA, 1987, 84, p. 6654-6658.
[15] : Corbett et al., 1975, Cancer Res, 35:2434-2439.

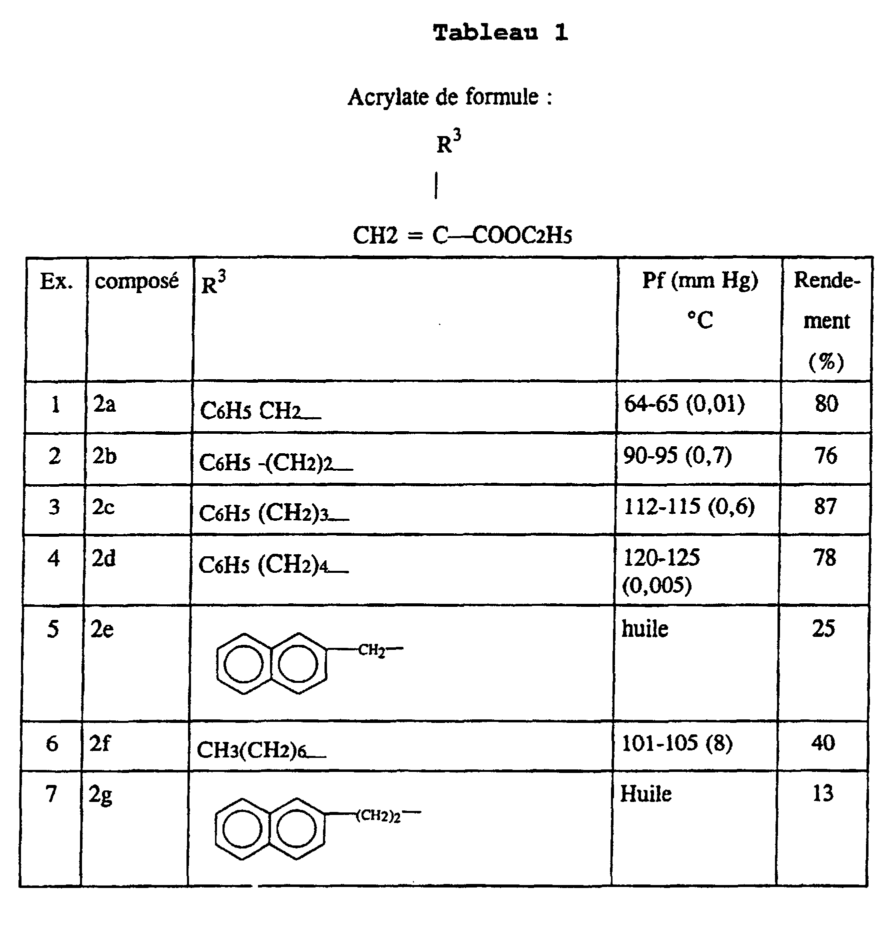

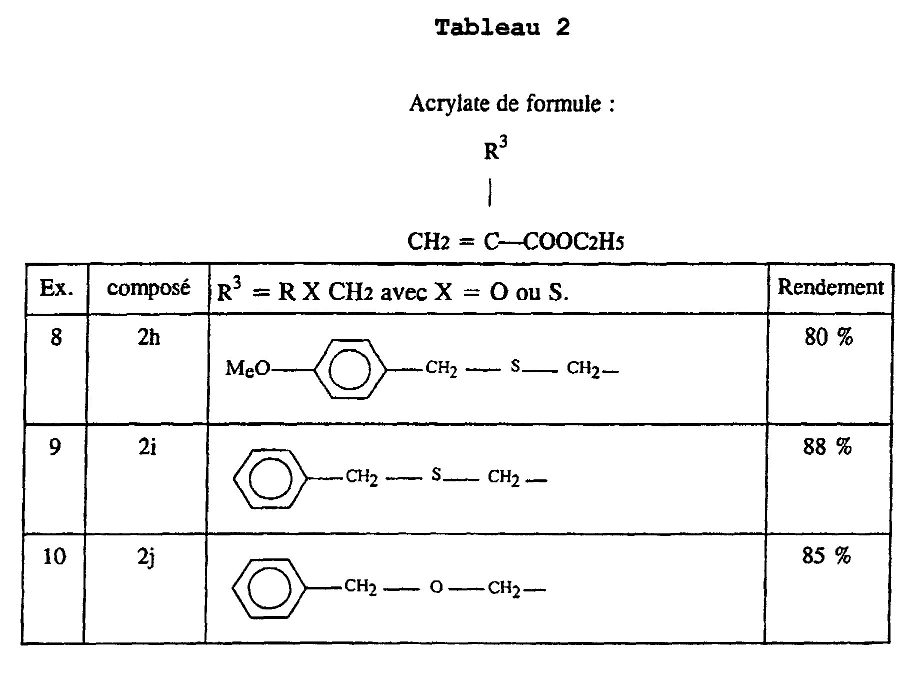

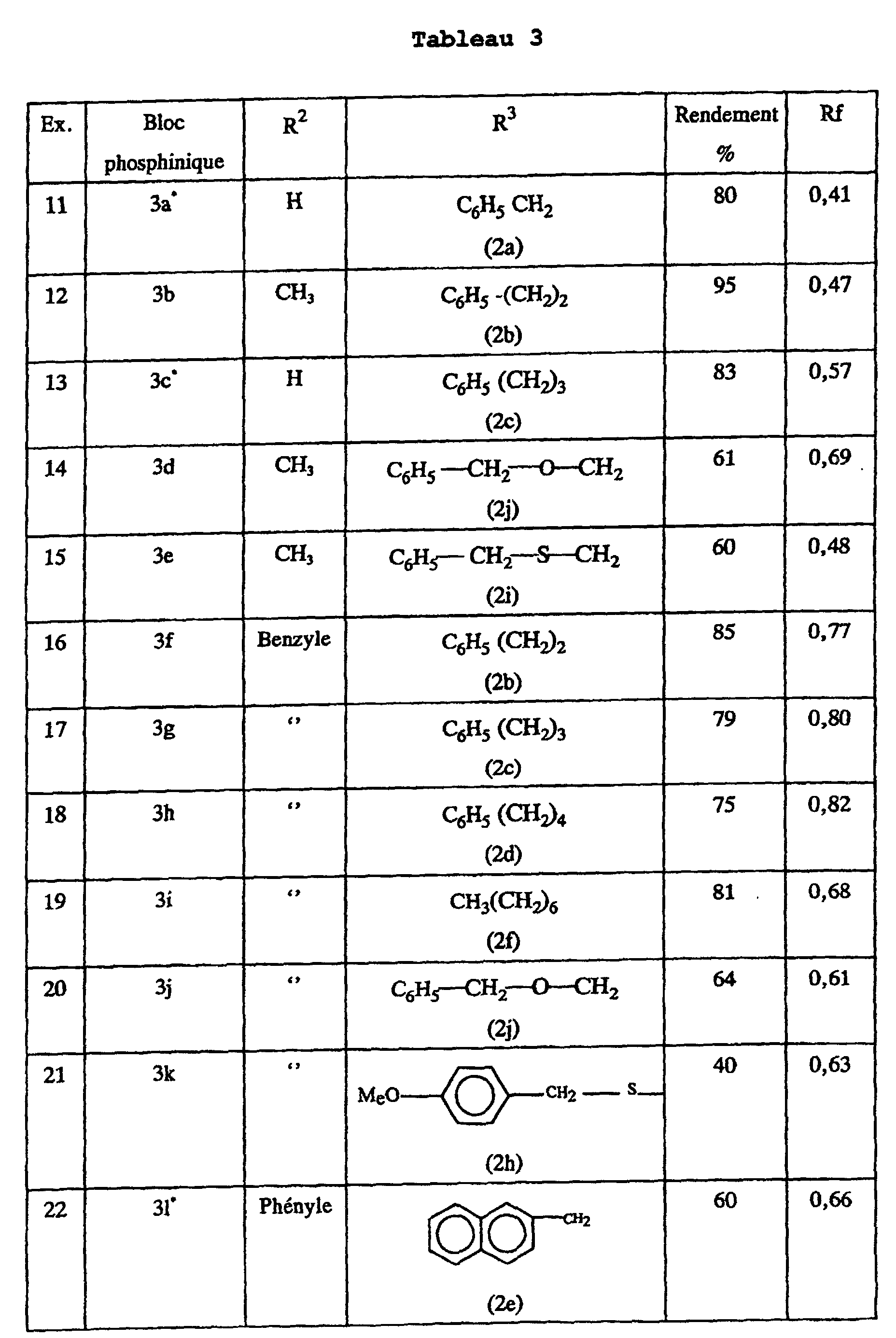

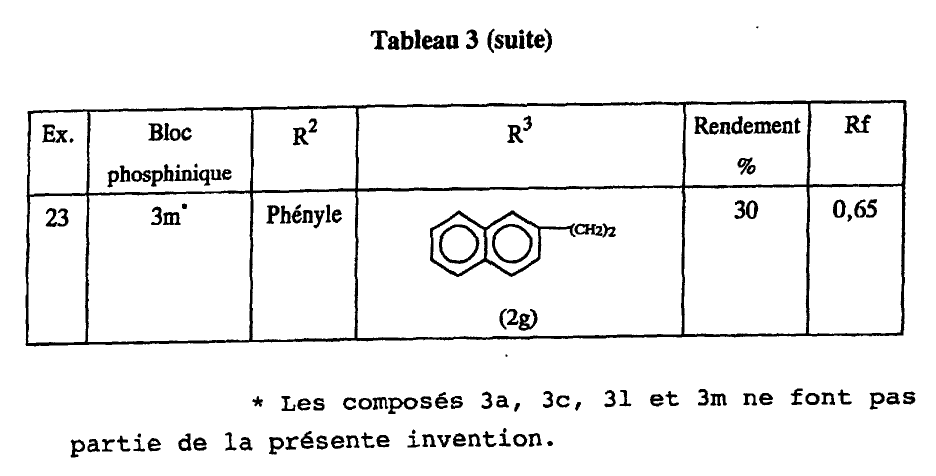

| **Composé de départ** | **Composé 4** | **Rendement** | **Rf**^{**(1)**} | **Composé 5** | **Rendement** | **Rf**^{**(2)**} |
|---|---|---|---|---|---|---|
| 3a | 4a | 95 | 0,70 | 5a | 80 | 0,53⁽¹⁾ |
| 3b | 4b | 97 | 0,72 | 5b | 81 | 0,59⁽¹⁾ |
| 3c | 4c | 97 | 0,73 | 5c | 85 | 0,21 |
| 3d | 4d | 93 | 0,77 | 5d | 90 | 0,42⁽¹⁾ |
| 3e | 4e | 72 | 0,72 | 5e | 92 | 0,50⁽¹⁾ |
| 3f | 4f | 89 | 0,61 | 5f | 95 | 0,42 |
| 3g | 4g | 75 | 0,66 | 5g | 97 | 0,42 |
| 3h | 4h | 95 | 0,63 | 5h | 95 | 0,44 |
| 3i | 4i | 96 | 0,78 | 5i | 78 | 0,56 |
| 3j | 4j | 91 | 0,71 | 5j | 80 | 0,26 |
| 3k | 4k | 93 | 0,66 | 5k | 94 | 0,46 |
| 3l | 4l | 91 | 0,53 | 5l | 92 | 0,38 |
| 3m | 4m | 95 | 0,57 | 5m | 86 | 0,35 |

| | | | | | | |
|---|---|---|---|---|---|---|
| ⁽¹⁾ dans le mélange hexane/acétate d'éthyle/acide acétique (3:3:0,2) | | | | | | |
| ⁽²⁾ dans le mélange chloroforme/méthanol (9,5: 0,5). | | | | | | |

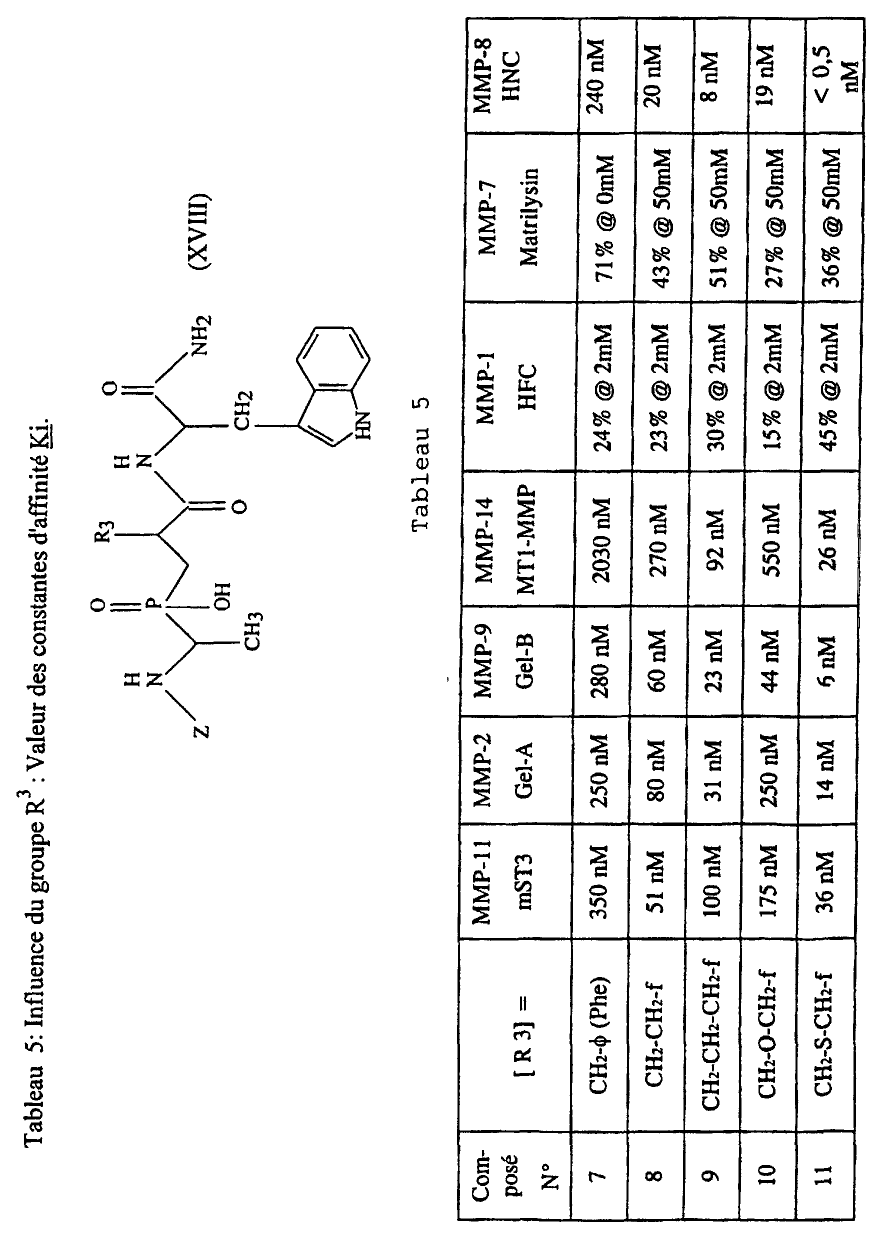

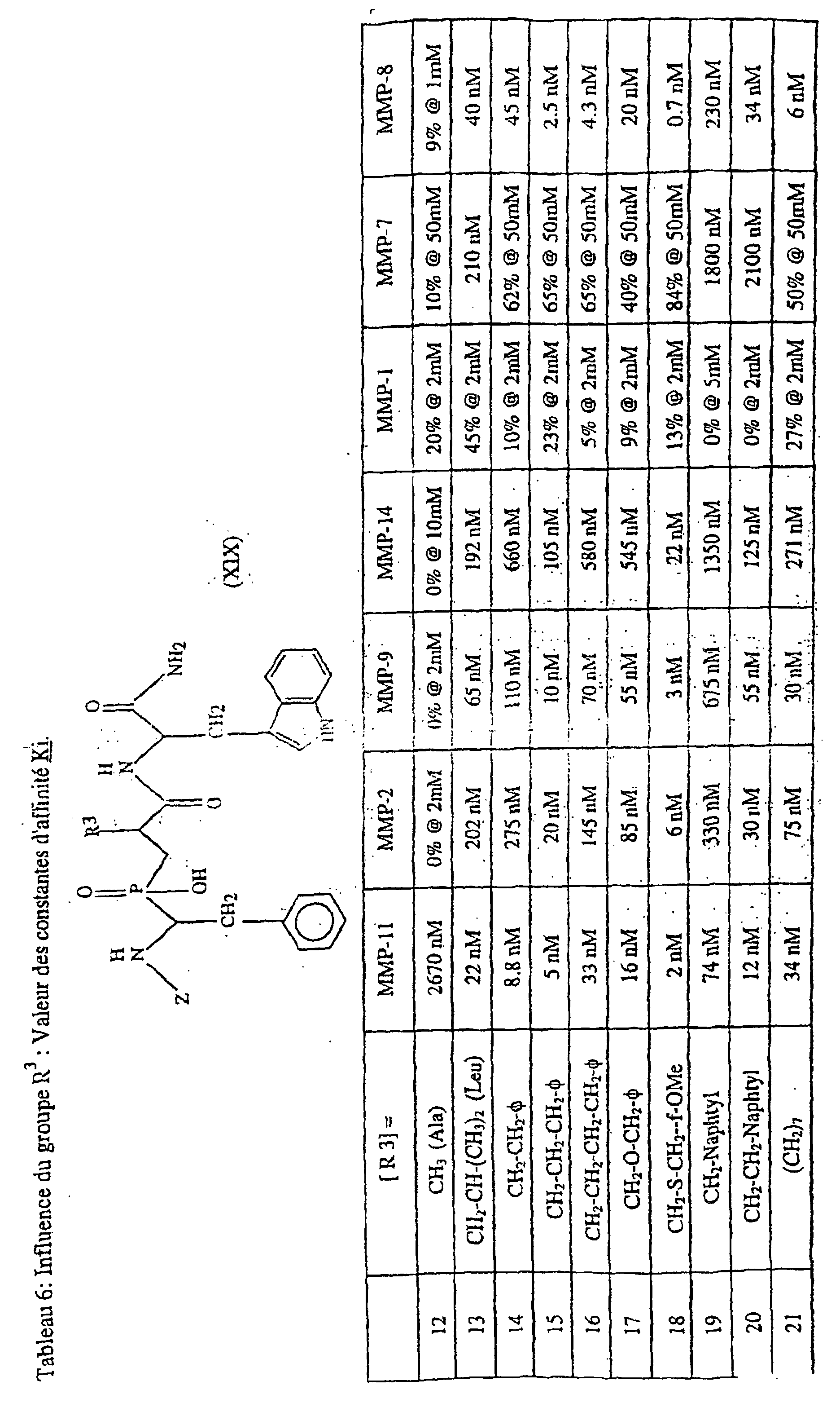

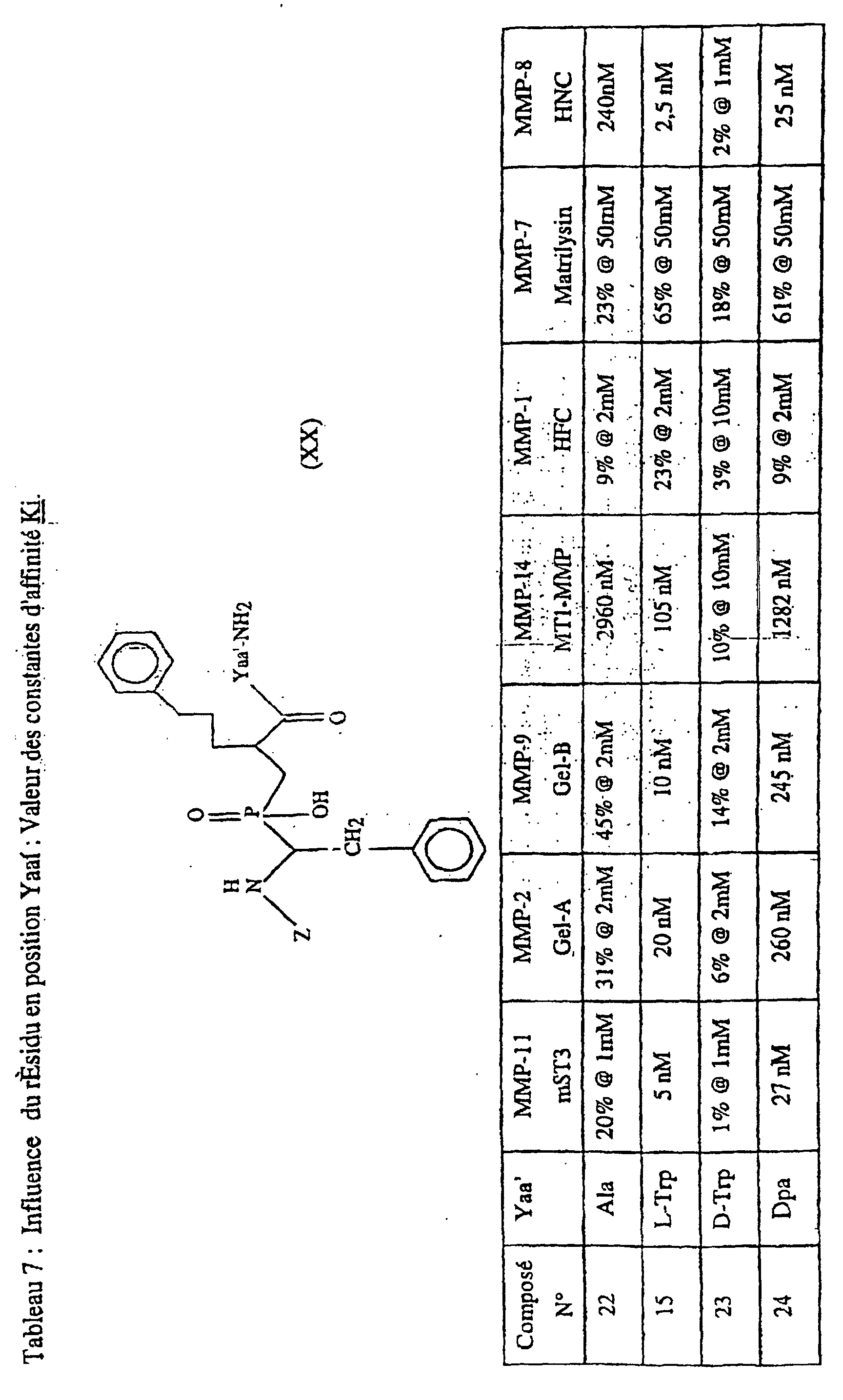

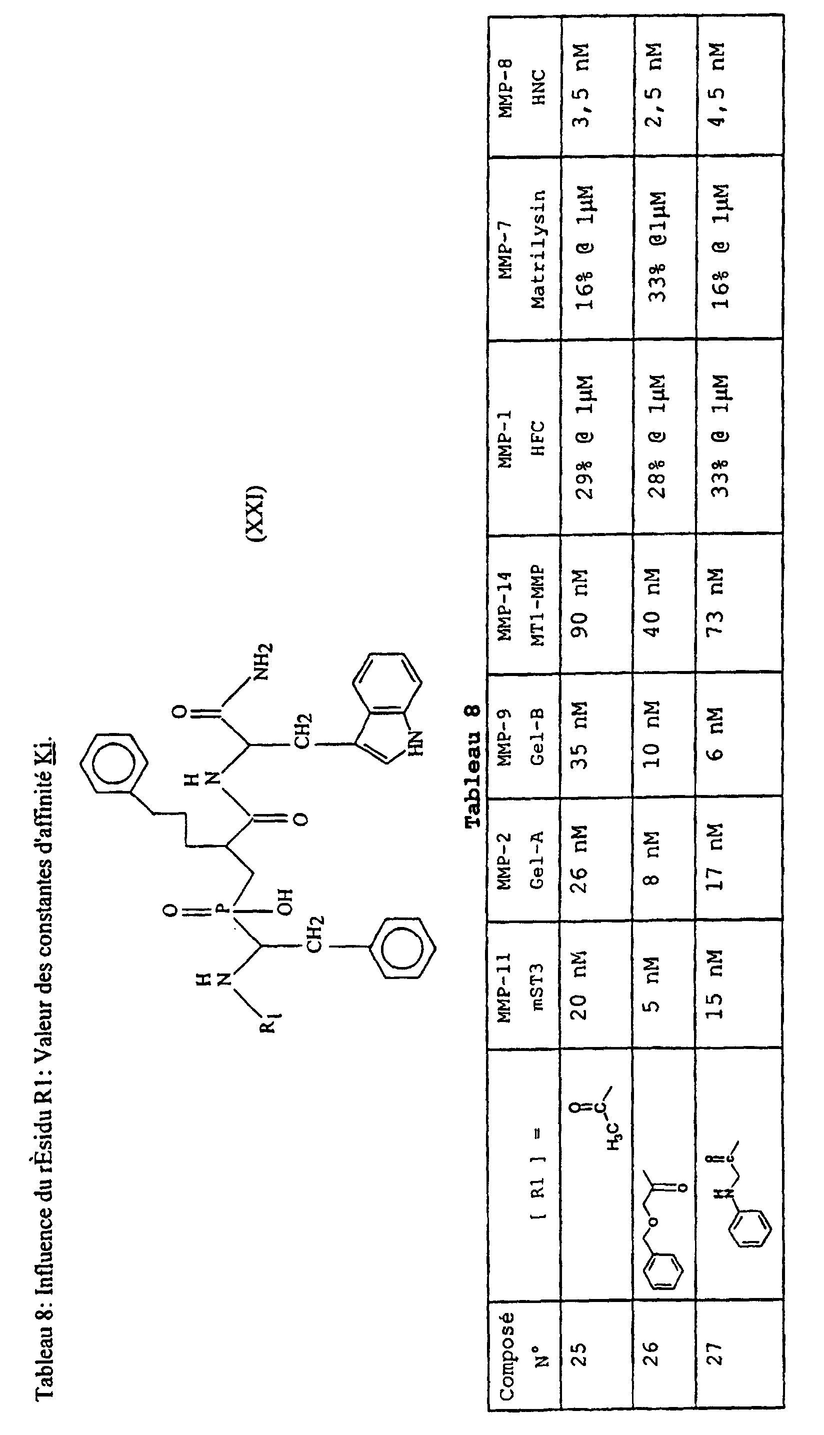

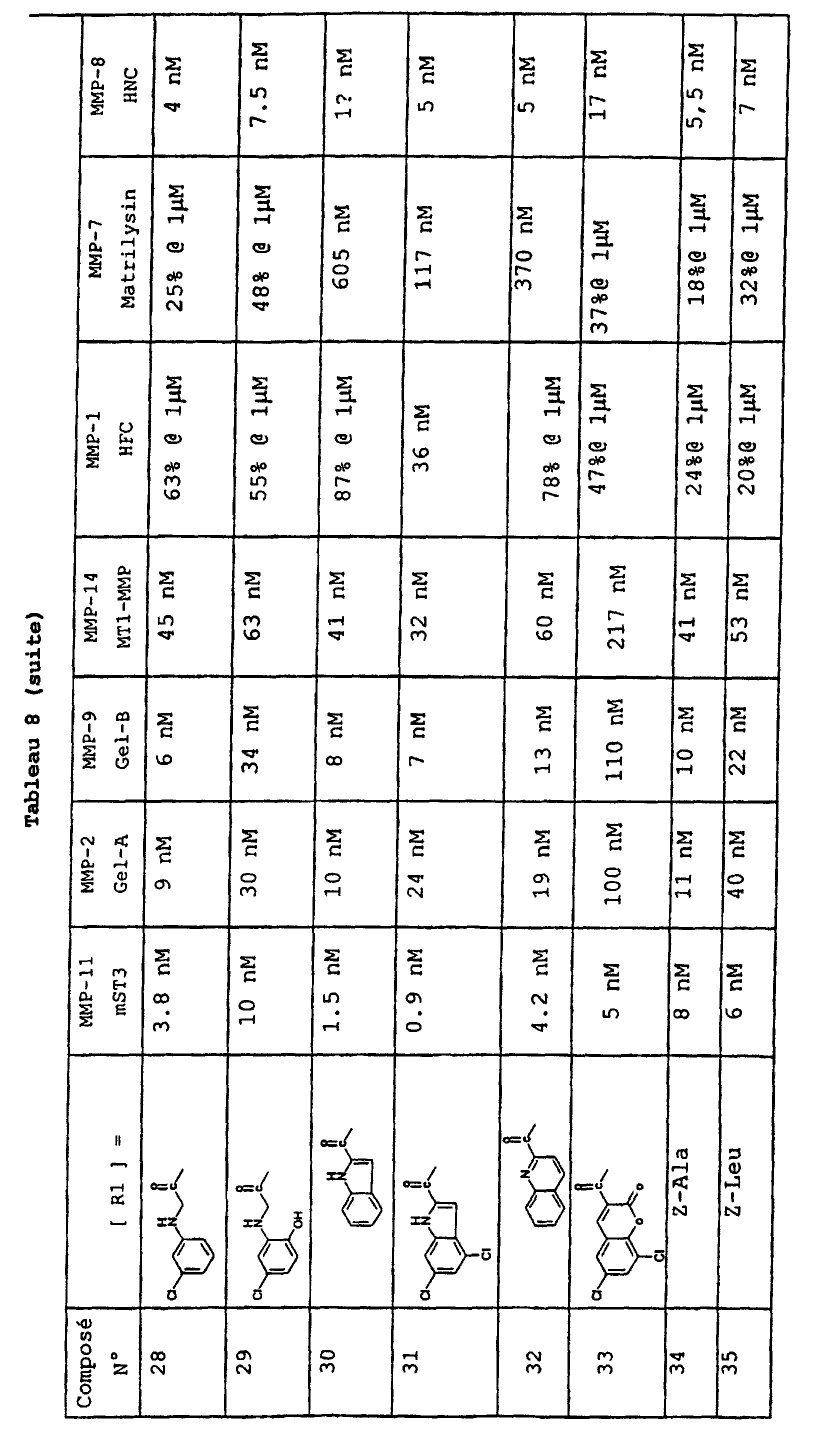

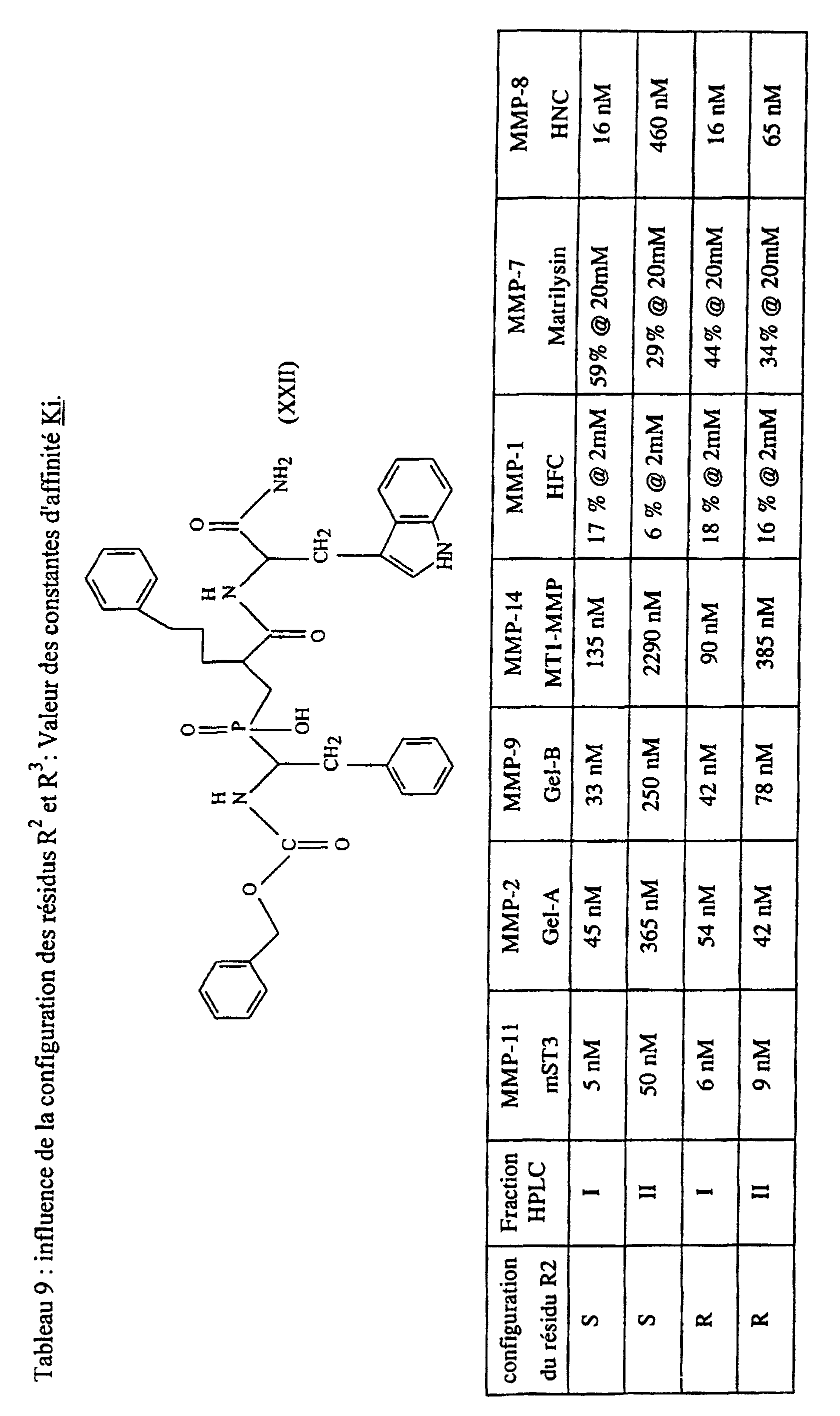

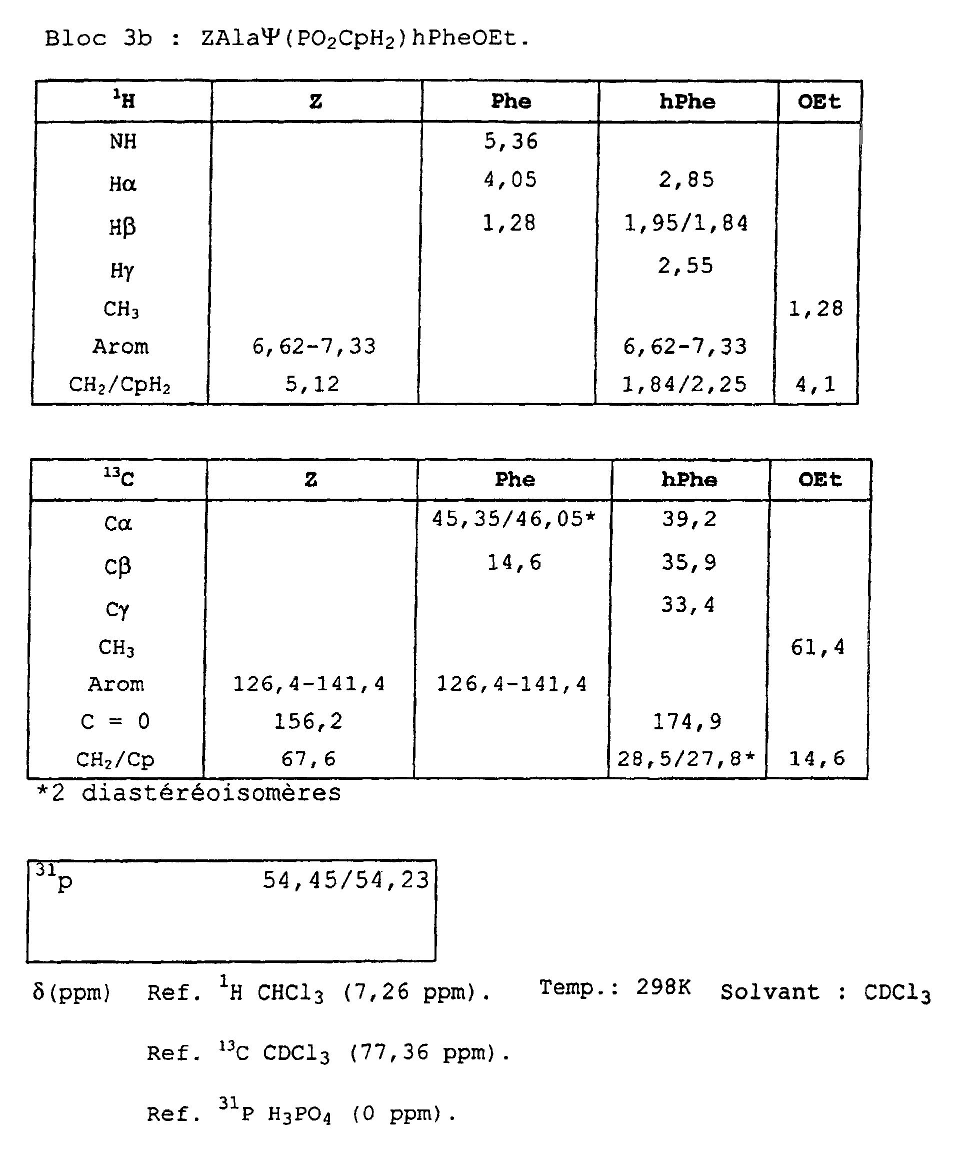

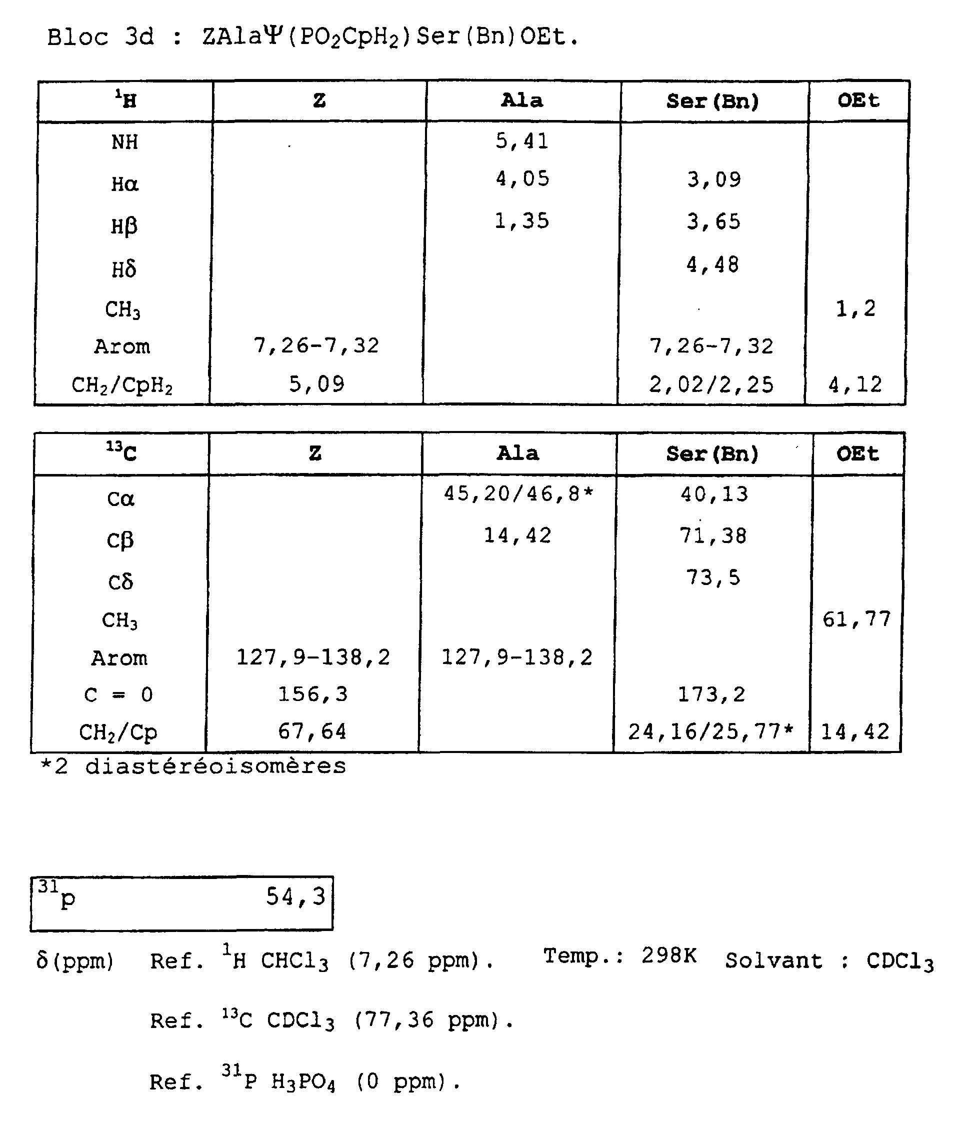

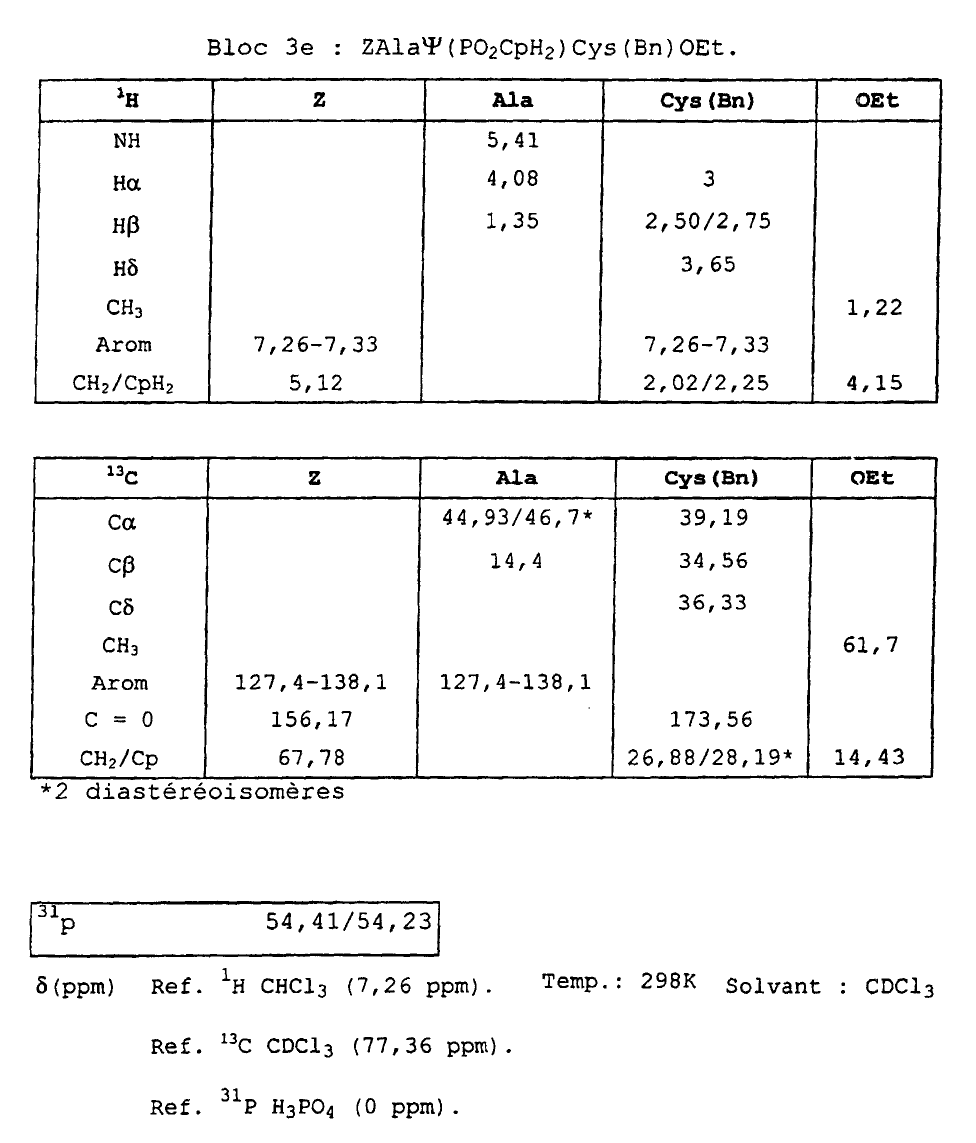

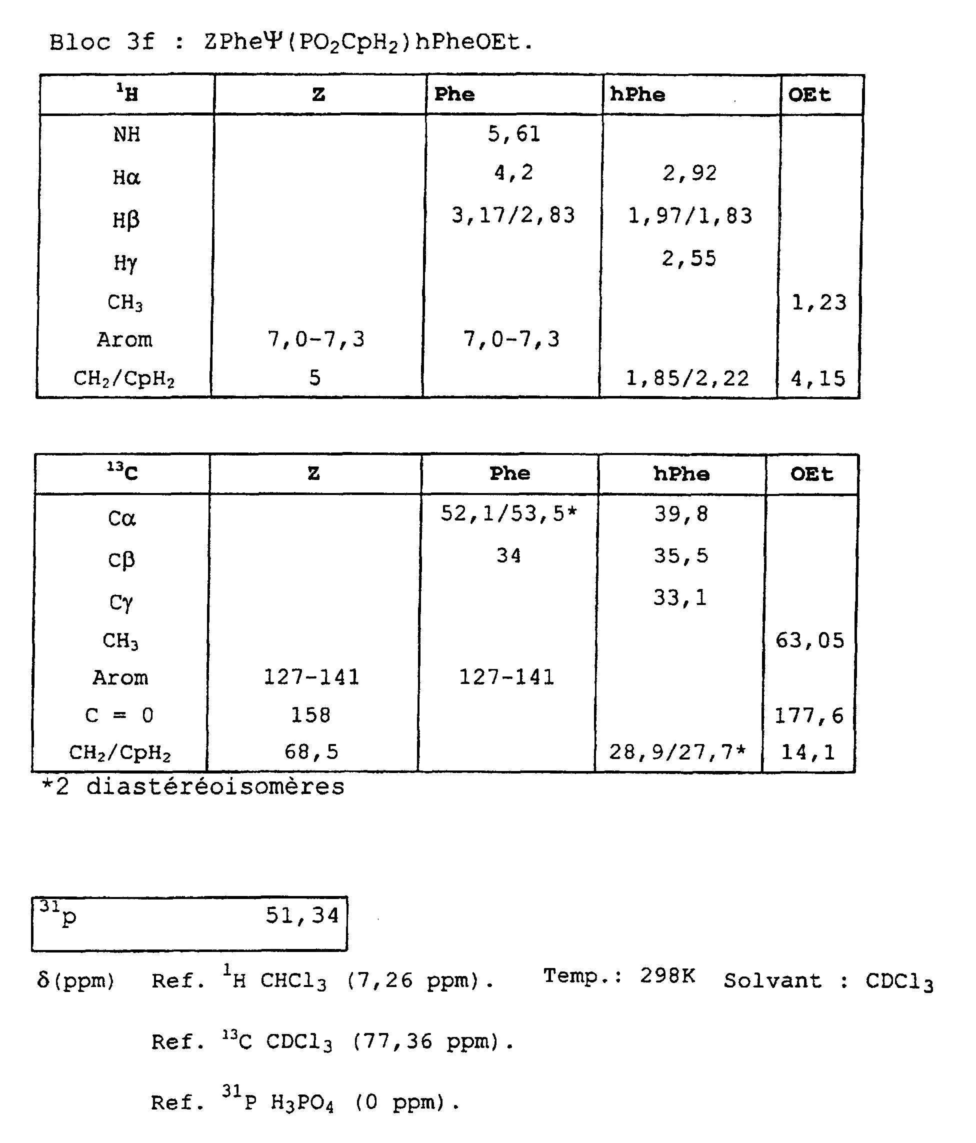

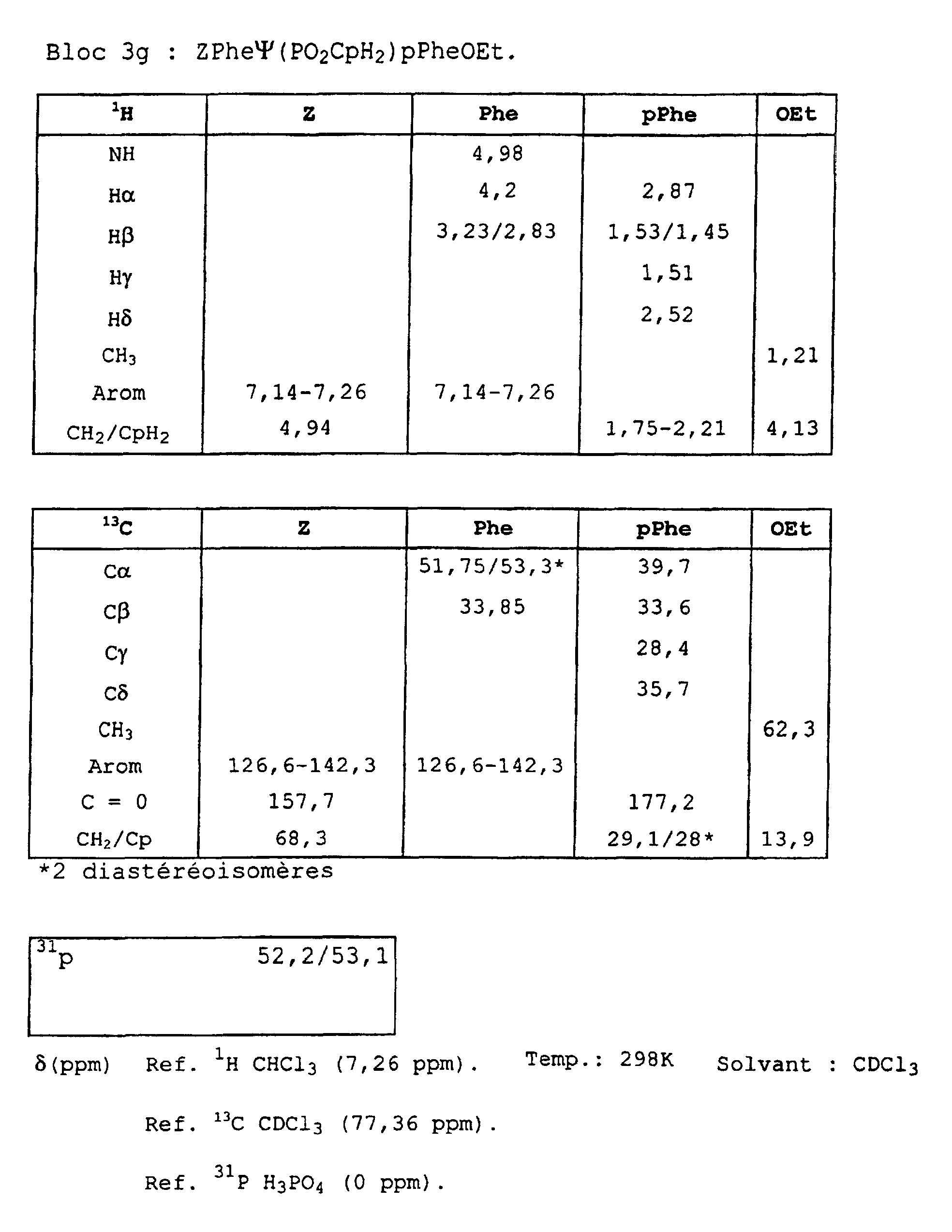

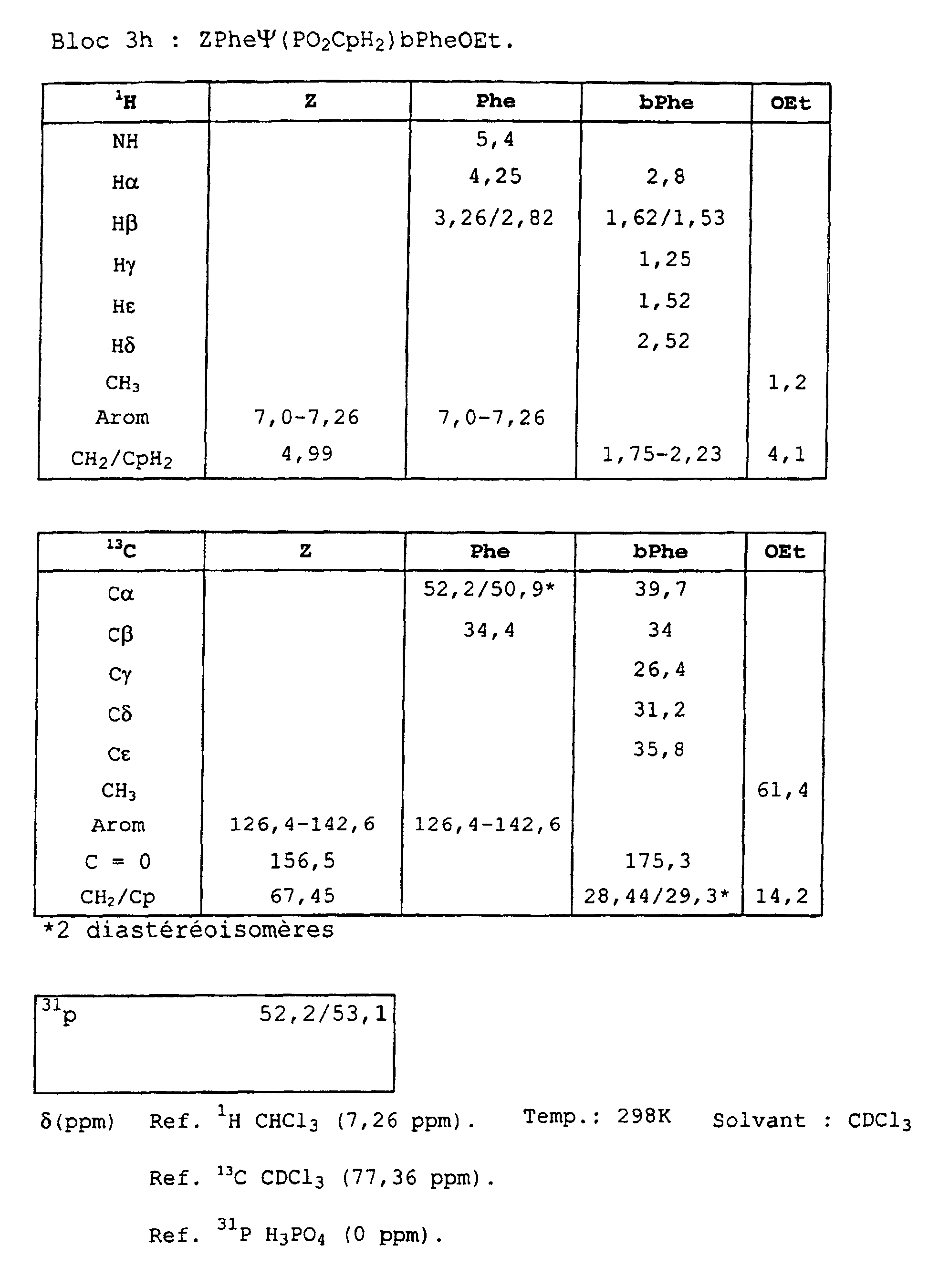

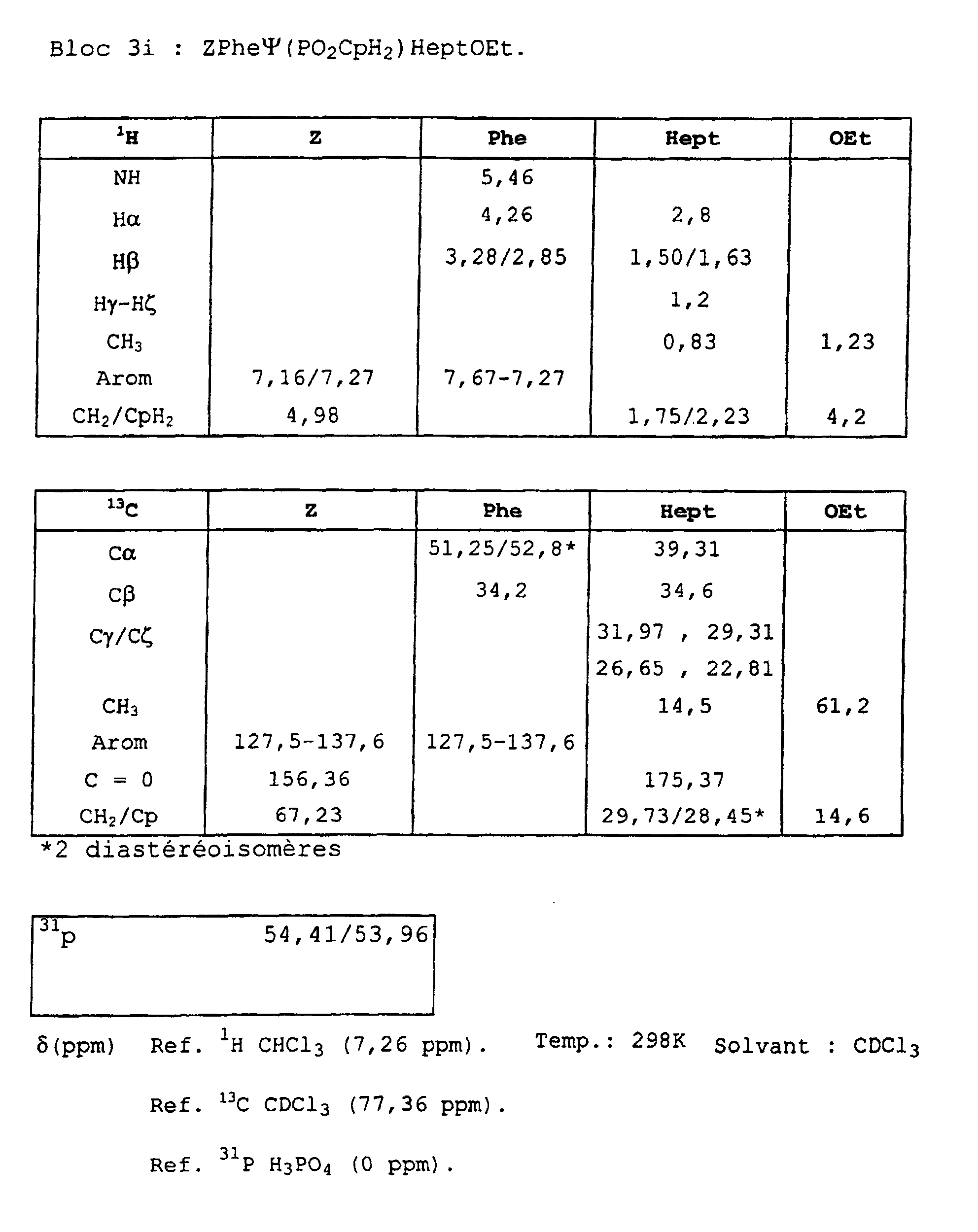

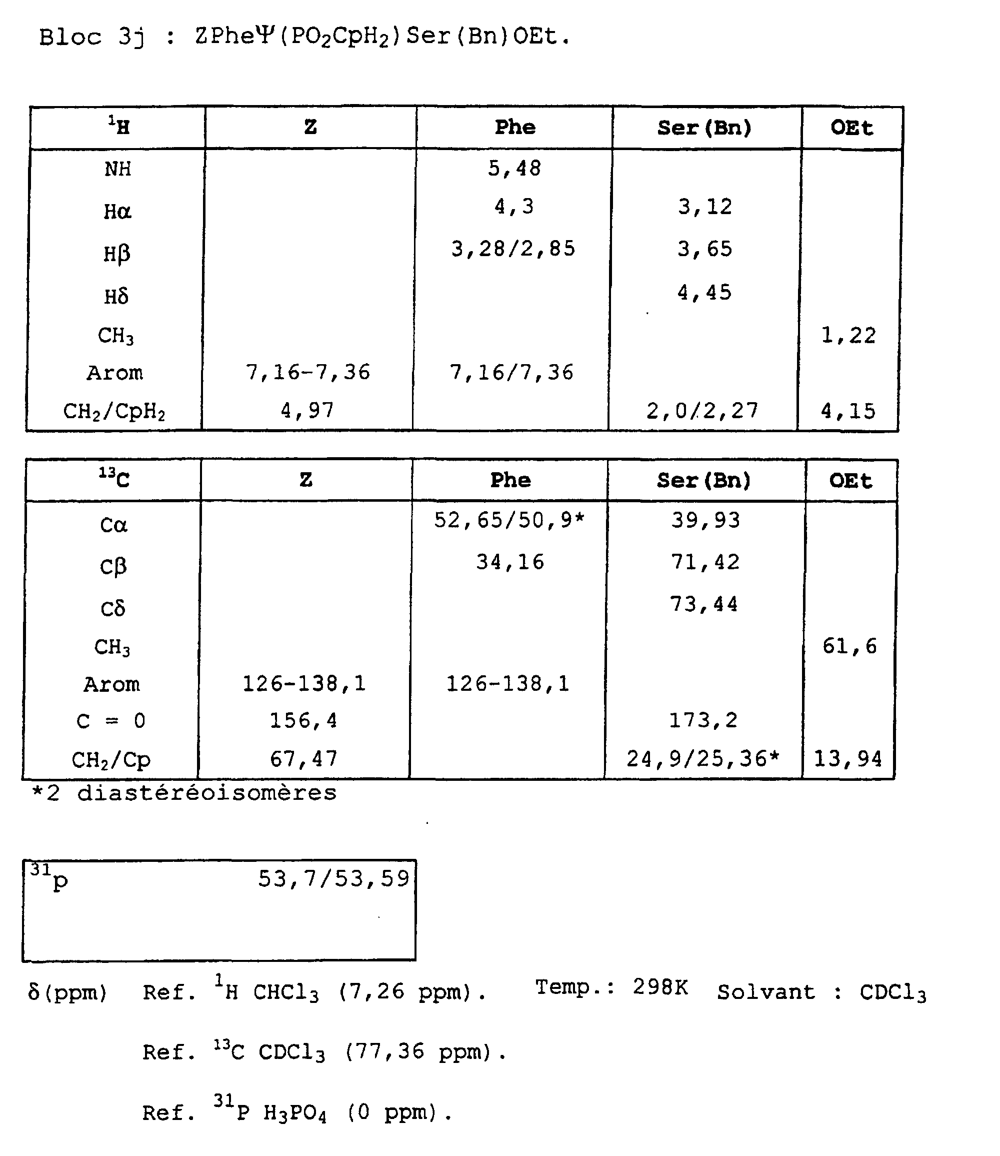

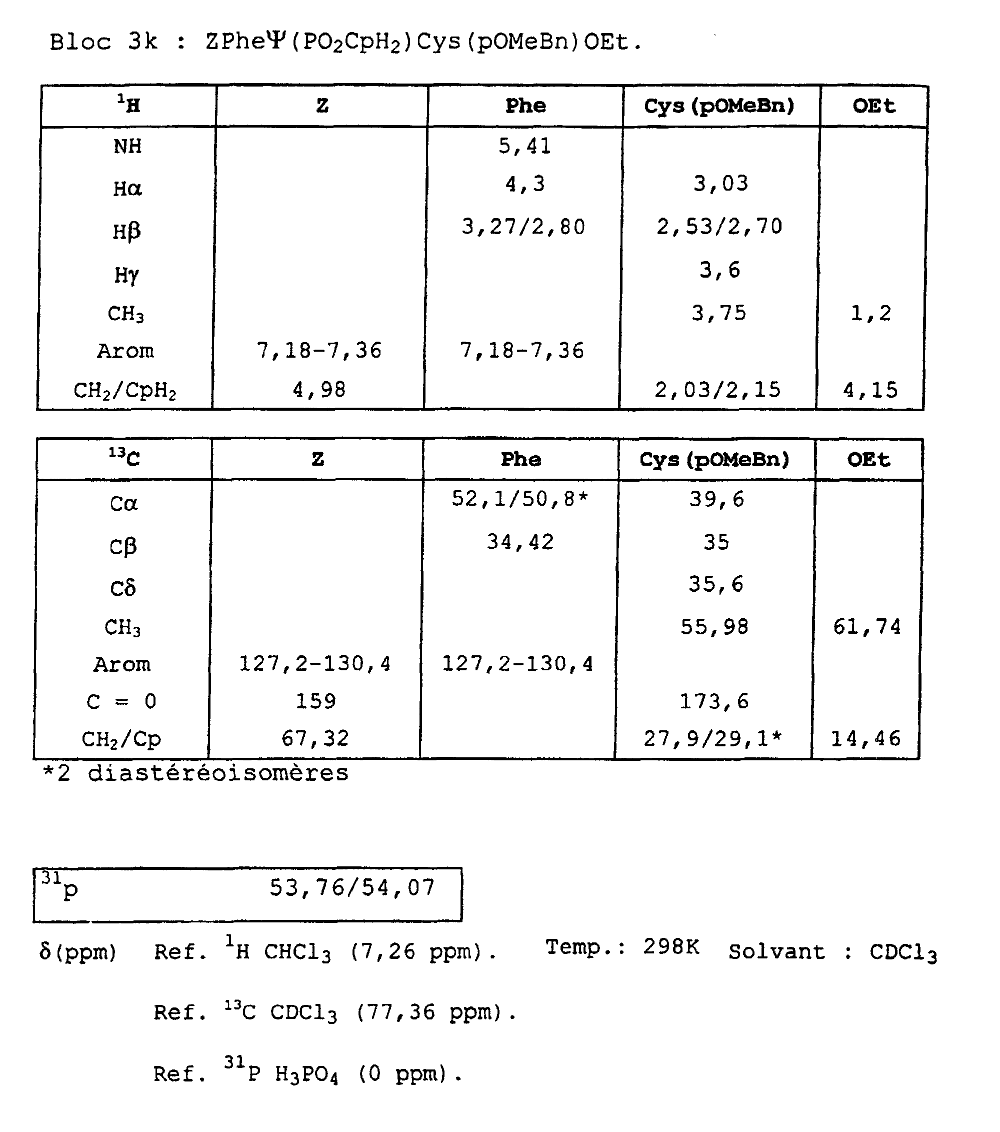

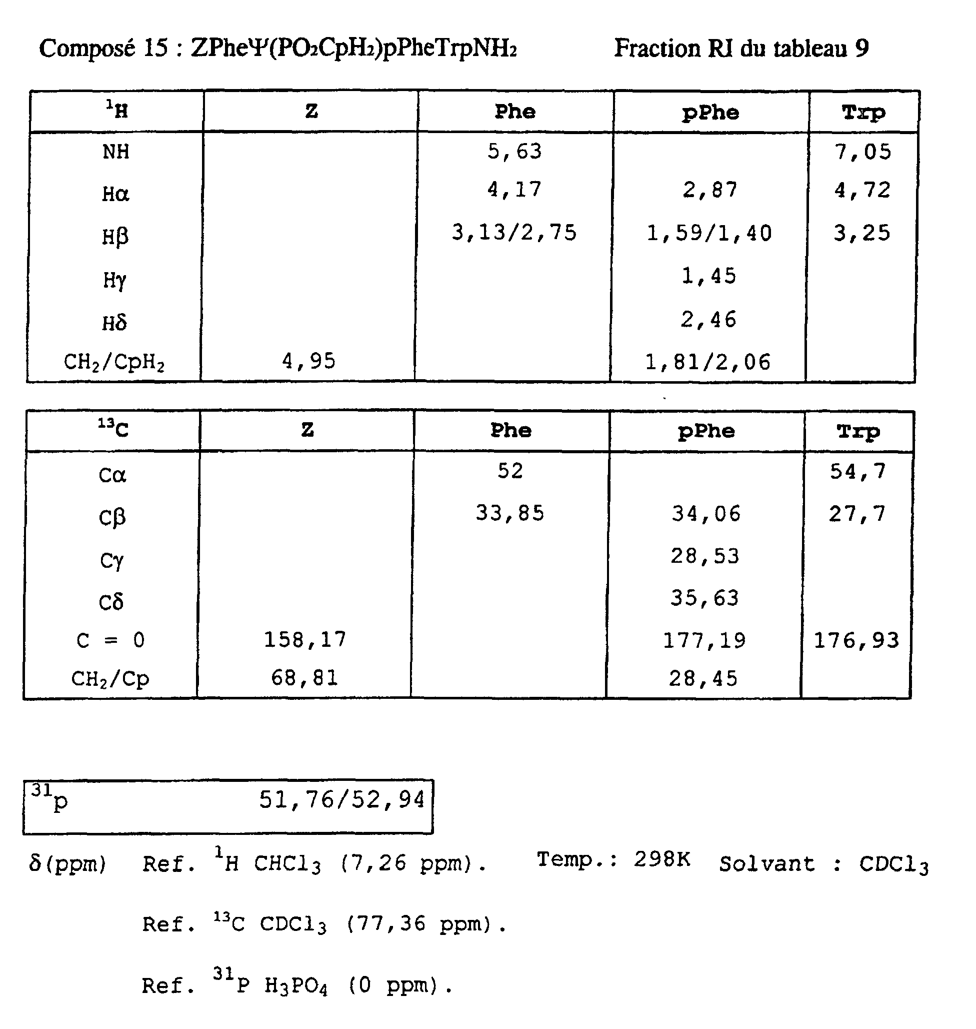

## Revendications

1. Pseudo-peptide phosphinique de formule : 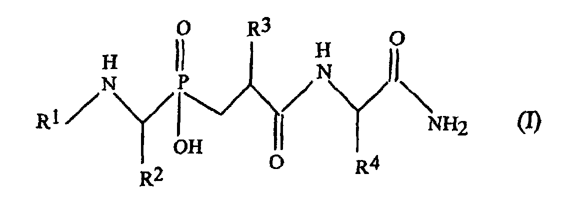 dans laquelle
- R¹ est un groupe bloquant une fonction aminé, choisi parmi les groupes t-butoxycarbonyle, benzyloxycarbonyle, cinnamoyle, pivaloyle, N-fluorénylméthoxycarbonyle, acétyle, benzyloxyacétyle, phénymaminoacétyle, (m-chlorophényl) aminoacétyle, (2-hydroxy-5-chloro-phényl)aminoacétyle, indolyl-2-carbonyle, 4, 6-dichloro indolyl-2-carbonyle, quinolyl-2-carbonyle et 1-oxa-2,4-dichloro-7-naphtalène carbonyle, ou un résidu d'aminoacide ou de peptide dont la fonction amine terminale est bloquée par un groupe bloquant choisi parmi les groupes t-butoxycarbonyle, benzyloxycarbonyle, cinnamoyle, pivaloyle, N-fluorénylméthoxycarbonyle, acétyle, benzyloxyacétyle, phénymaminoacétyle, (m-chlorophényl) aminoacétyle, (2-hydroxy-5-chloro-phényl)aminoacétyle, indolyl-2-carbonyle, 4,6-dichloro indolyl-2-carbonyle, quinolyl-2-carbonyle et 1-oxa-2,4-dichloro-7-naphtalène carbonyle,
- R² représente la chaîne latérale d'un acide aminé naturel ou non naturel, choisi parmi l'alanine, l'arginine, l'asparagine, l'acide aspartique, la cystéine, la glutamine, l'acide glutamique, la glycine, l'histidine, l'isoleucine, la leucine, la norleucine, la lysine, la méthionine, la phénylalanine, la proline, l'hydroxyproline, la sérine, la thréonine, le tryptophane, la thyrosine, la valine, la nitrophénylalanine, l'homoarginine, la thiazolidine et la déshydroproline,
- R³ représente :
1) la chaîne latérale d'un acide aminé naturel sauf Gly et Ala, non substituée ou substituée par un groupe aryle,
2) un groupe aralkyle, ou
3) un groupe alkyle d'au moins 3 atomes de carbone, et
- R⁴ représente un groupe dinitrobenzyle ou une chaîne latérale d'acide aminé naturel ou non naturel, choisi parmi l'alanine, l'arginine, l'asparagine, l'acide aspartique, la cystéine, la glutamine, l'acide glutamique, la glycine, l'histidine, l'isoleucine, la leucine, la norleucine, la lysine, la méthionine, la phénylalanine, la proline, l'hydroxyproline, la sérine, la thréonine, le tryptophane, la thyrosine, la valine, la nitrophénylalanine, l'homoarginine, la thiazolidine et la déshydroproline.

2. Pseudo-peptide selon la revendication 1, dans lequel R² représente le groupe méthyle ou benzyle.

3. Pseudo-peptide selon la revendication 1, dans lequel R³ représente la chaîne latérale d'un acide aminé choisi parmi Phe et Leu ou un résidu Ser ou Cys dont la chaîne latérale est substituée par un groupe arylakyle.

4. Pseudo-peptide selon la revendication 1, dans lequel R³ est un groupe aralkyle choisi parmi les groupes de formule : 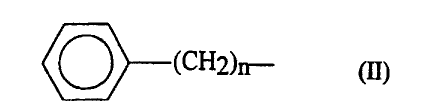 avec n étant un nombre entier de 1 à 4, et de formule : 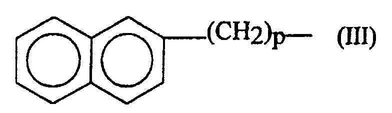 avec p égal à 1 ou 2.

5. Pseudo-peptide selon la revendication 1 dans lequel R³ est un groupe répondant à l'une des formules suivantes : 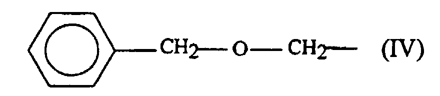 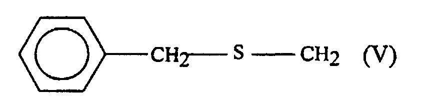
(CH₃)₂-CH-CH₂- (VI)

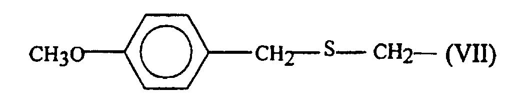

6. Pseudo-peptide selon la revendication 1, dans lequel R³ est le groupe de formule :

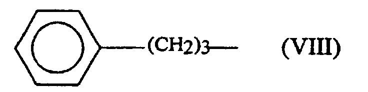

7. Pseudo-peptide selon l'une quelconque des revendications 1 à 6, dans lequel R⁴ représente le groupe de formule :

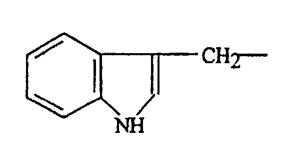

8. Pseudo-peptide selon l'une quelconque des revendications 1 à 6, dans lequel R¹ représente un groupe choisi parmi les groupes acétyle, benzyloxyacétyle, phénylaminoacétyle, m-chlorophényl)aminoacétyle, (2-hydroxy-5-chloro-phényl) amino acétyle, indolyl-2-carbonyle, 4,6-dichloro-indolyl-2-carbonyle, quinolyl-2-carbonyle et 1-oxa-2, 4-dichloro-7-naphtalène carbonyle.

9. Pseudo-peptide selon la revendication 1 qui répond à la formule : 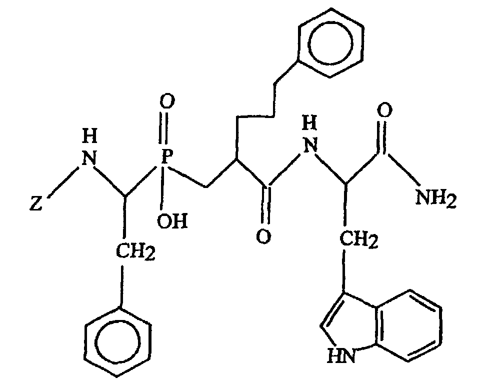 dans laquelle Z est le groupe benzyloxycarbonyle.

10. Pseudo-peptide selon la revendication 9, dans lequel le carbone asymétrique de l'ensemble : 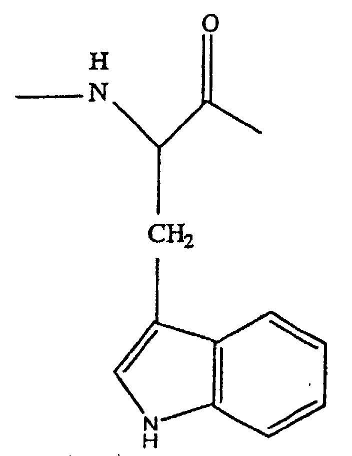 a la configuration L.

11. Composition pharmaceutique pour utilisation comme inhibiteur d'au moins une métalloprotéase à zinc matricielle, comprenant au moins un pseudo-peptide selon l'une quelconque des revendications 1 à 10.

12. Composition selon la revendication 11 dans laquelle la métalloprotéase à zinc matricielle est choisie parmi les MMP-2, MMP-8, MMP-9 et MMP-11.

13. Composition pharmaceutique selon l'une quelconque des revendications 11 et 12, pour le traitement d'une maladie **caractérisée par** la surexpression des protéases matricielles.

14. Composition selon la revendication 13, pour le traitement du cancer.

15. Utilisation d'un pseudo-peptide phosphinique selon l'une quelconque des revendications 1 à 10 pour la fabrication d'un médicament inhibant au moins une métalloprotéase à zinc matricielle.

16. Utilisation selon la revendication 15, dans laquelle la métalloprotéase à zinc matricielle est choisie parmi les MMP-2, MMP-8, MMP-9 et MMP-11.

17. Utilisation selon la revendication 15 ou 16, dans laquelle le médicament est destiné au traitement d'une maladie **caractérisée par** la surexpression des protéases matricielles.

18. Utilisation selon la revendication 17, dans laquelle la maladie est le cancer.

## Patentansprüche

1. Phosphinsäurepseudopeptid der Formel 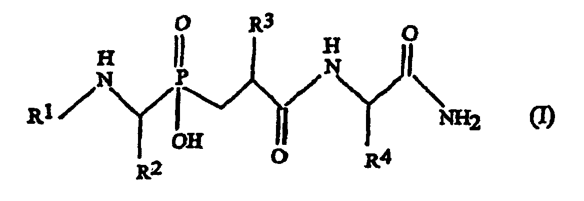 worin bedeuten:
- R¹ eine eine Aminfunktion blockierende Gruppe, ausgewählt unter den t-Butoxycarbonyl-, Benzyloxycarbonyl-, Cinnamoyl-, Pivaloyl-, N-Fluorenylmethoxycarbonyl-, Acetyl-, Benzyloxyacetyl-, Phenylaminoacetyl-, (m-Chlorophenyl)aminoacetyl-, (2-Hydroxy-5-chlorophenyl)aminoacetyl-, Indolyl-2-carbonyl-, 4,6-Dichloroindolyl-2-carbonyl-, Chinolyl-2-carbonyl- und 1-Oxa-2,4-dichloro-7-naphthalincarbonyl-Gruppen oder den Rest einer Aminosäure oder einen Peptidrest, dessen terminale Aminfunktion blockiert ist durch eine Blockierungsgruppe, ausgewählt unter den t-Butoxycarbonyl-, Benzyloxycarbonyl-, Cinnamoyl-, Pivaloyl-, N-Fluorenyl-methoxycarbonyl-, Acetyl-, Benzyloxyacetyl-, Phenylaminoacetyl-, (m-Chlorophenyl)aminoacetyl-, (2-Hydroxy-5-chlorophenyl)aminoacetyl-, Indolyl-2-carbonyl-, 4,6-Dichloroindolyl-2-carbonyl-, Chinolyl-2-carbonyl- und 1-Oxa-2,4-dichloro-7-naphthalincarbonyl-Gruppen,
- R² die Seitenkette einer natürlichen oder nicht-natürlichen Aminosäure, ausgewählt aus der Gruppe Alanin, Arginin, Asparagin, Asparaginsäure, Cystein, Glutamin, Glutaminsäure, Glycin, Histidin, isoleucin, Leucin, Norleucin, Lysin, Methionin, Phenylalanin, Prolin, Hydroxyprolin, Serin, Threonin, Tryptophan, Thyrosin, Valin, Nitrophenylalanin, Homoarginin, Thiazolidin und Dehydroprolin,
- R³
1) die Seitenkette einer natürlichen Aminosäure, ausgenommen Gly und Ala, die unsubstituiert oder durch eine Arylgruppe substituiert ist,
2) eine Aralkylgruppe, oder
3) eine Alkylgruppe mit mindestens 3 Kohlenstoffatomen, und
- R⁴ eine Dinitrobenzylgruppe oder eine Seitenkette einer natürlichen oder nicht-natürlichen Aminosäure, ausgewählt aus der Gruppe Alanin, Arginin, Asparagin, Asparaginsäure, Cystein, Glutamin, Glutaminsäure, Glycin, Histitidin, Isoleucin, Leucin, Norleucin, Lysin, Methionin, Phenylalanin, Prolin, Hydroₓyprolin, Serin, Threonin, Tryptophan, Thyrosin, Valin, Nitrophenylalanin, Homoarginin, Thiazolidin und Dehydroprolin.

2. Pseudopeptid nach Anspruch 1, worin R² steht für die Methyl- oder Benzylgruppe.

3. Pseudopeptid nach Anspruch 1, worin R³ steht für die Seitenkette einer Aminosäure, ausgewählt unter Phe und Leu oder für einen Rest Ser oder Cys, dessen Seitenkette durch eine Arylalkylgruppe substituiert ist.

4. Pseudopeptid nach Anspruch 1, worin R³ steht für eine Aralkylgruppe, ausgewählt unter den Gruppen der Formel: 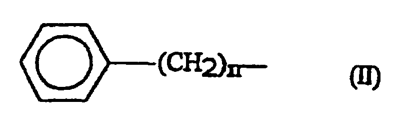 worin n steht für eine ganze Zahl von 1 bis 4, und der Formel: 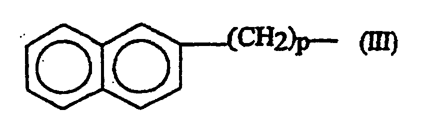 worin p steht für die Zahl 1 oder 2.

5. Pseudopeptid nach Anspruch 1, worin R³ steht für eine Gruppe, die einer der folgenden Formeln entspricht: 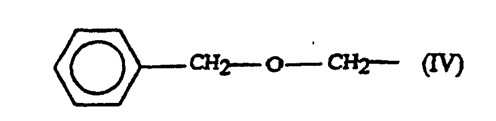 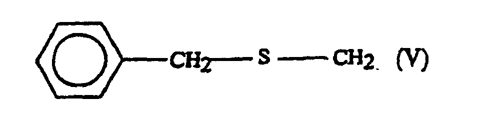
(CH₃)₂-CH-CH₂- (VI)

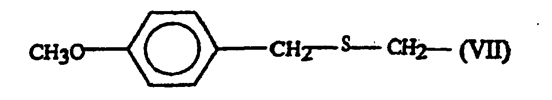

6. Pseudopeptid nach Anspruch 1, in der R³ steht für die Gruppe der Formel:

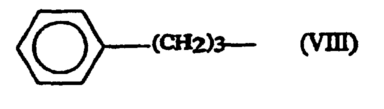

7. Pseudopeptid nach einem der Ansprüche 1 bis 6, worin R⁴ steht für eine Gruppe der Formel:

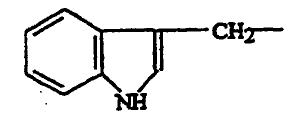

8. Pseudopeptid nach einem der Ansprüche 1 bis 6, worin R¹ steht für eine Gruppe, ausgewählt unter den Acetyl-, Benzytoxyacetyl-, Phenylaminoacetyl-, m-Chlorophenyl)aminoacetyl-, (2-Hydroxy-5-chloro-phenyl)amlnoacetyl-, Indolyl-2-carbonyl-, 4,6-Dichloro-indolyl-2-carbonyl-, Chinolyl-2-carbonyl- und 1-Oxa-2,4-dichloro-7-naphthalincarbonyl-Gruppen.

9. Pseudopeptid nach Anspruch 1, das der Formel entspricht: 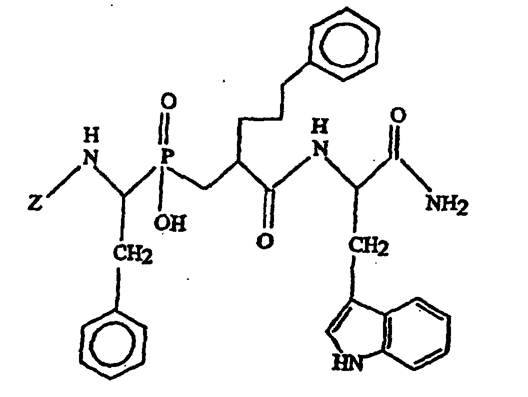 worin Z steht für die Benzyloxycarbonylgruppe.

10. Pseudopeptid nach Anspruch 9, worin das asymmetrische Kohlenstoffatom des Ganzen die Konfiguration L hat

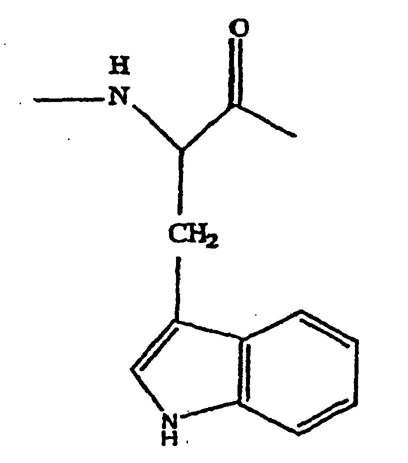

11. Pharmazeutische Zusammensetzung zur Verwendung als Inhibitor mindestens einer Metalloprotease mit Zinkmatrix, die umfasst mindestens ein Pseudopeptid nach einem der Ansprüche 1 bis 10.

12. Zusammensetzung nach Anspruch 11, in der die Metallprotease mit Zinkmatrix ausgewählt ist unter MMP-2, MMP-8, MMP-9 und MMP-11.

13. Pharmazeutische Zusammensetzung nach einem der Ansprüche 11 und 12 für die Behandlung einer Erkrankung, die durch die Überexpression von Matrizenproteasen charakterisiert ist.

14. Zusammensetzung nach Anspruch 13 für die Behandlung von Krebs.

15. Verwendung eines Phosphinsäurepseudopeptids nach einem der Ansprüche 1 bis 10 zur Herstellung eines Arzneimittels, das mindestens eine Metallprotease mit Zinkmatrix inhibiert.

16. Verwendung nach Anspruch 15, bei der die Metallprotease mit Zinkmatrix ausgewählt ist unter MMP-2, MMP-8, MMP-9 und MMP-11.

17. Verwendung nach Anspruch 15 oder 16, bei der das Arzneimittel zur Behandlung einer Erkrankung bestimmt ist, die durch die Überexpression von Matrizenproteasen charakterisiert ist.

18. Verwendung nach Anspruch 17, bei der die Krankheit Krebs ist.

## Claims

1. Phosphinic pseudo-peptide of formula: 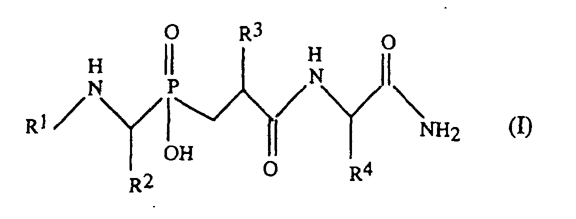 Wherein
- R¹ is a group inhibiting an amine function chosen from among the following groups: t-butoxycarbonyl, benzyloxycarbonyl, cinnamyl, pivalyl, N-fluorenylmethoxycarbonyl, acetyl, benzyloxyacetyl, phenylaminoacetyl, (m-chlorophenyl) aminoacetyl, (2-hydroxy-5-chloro-phenyl) aminoacetyl, indolyl-2-carbonyl, 4,6-dichloro indolyl-2-carbonyl, quinolyl-2-carbonyl and 1-oxa-2,4-dichloro-7-naphthalene carbonyl, or a residue of amino acid or peptide, whose terminal amine function is inhibited by an inhibiting group chosen from among the following groups: t-butoxycarbonyl, benzyloxycarbonyl, cinnamyl, pivalyl, N-fluorenyl-methoxycarbonyl, acetyl, benzyloxyacetyl, phenylaminoacetyl, (m-chlorophenyl) aminoacetyl, (2-hydroxy-5-chloro-phenyl) aminoacetyl, indolyl-2-carbonyl, 4,6-dichloro indolyl-2-carbonyl, quinolyl-2-carbonyl and 1-oxa-2,4-dichloro-7-naphthalene carbonyl,
- R² represents the lateral chain of a natural or non-natural amino acid chosen from among alanine, arginine, asparagine, aspartic acid, cysteine, glutamine, glutamic acid, glycine, histidine, isoleucine, leucine, norleucine, lysin, methionine, phenylalanine, proline, hydroxyproline, serine, threonine, tryptophan, thyrosine, valine, nitrophenylalanine, homoarginine, thiazolidine and dehydroproline,
- R³ represents:
1) the lateral chain of a natural amino acid, excepting Gly and Ala, not substituted or substituted by an aryl group,
2) an aralkyl group, or
3) an alkyl group with at least three carbon atoms and
- R⁴ represents a dinitrobenzyl group or a lateral chain of a natural or non-natural amino acid chosen from among alanine, arginine, asparagine, aspartic acid, cysteine, glutamine, glutamic acid, glycine, histidine, isoleucine, leucine, norleucine, lysin, methionine, phenylalanine, proline, hydroxyproline, serine, threonine, tryptophan, thyrosine, valine, nitrophenylalanine, homoarginine, thiazolidine and dehydroproline.

2. Pseudo-peptide according to claim 1, wherein R² represents the methyl or benzyl group.

3. Pseudo-peptide according to claim 1, wherein R³ represents the lateral chain of an amino acid chosen from among Phe and Leu or a residue Ser or Cys, whose lateral chain is substituted by an aryl alkyl group.

4. Pseudo-peptide according to claim 1, wherein R³ is an aralkyl group chosen from among the groups of formula: 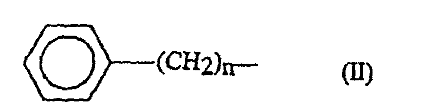 with n being an integer between 1 and 4, and formula: 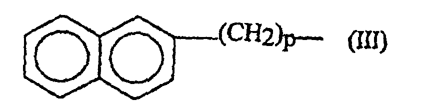 with p equal to 1 or 2.

5. Pseudo-peptide according to claim 1, wherein R³ is a group complying with one of the following formulas: 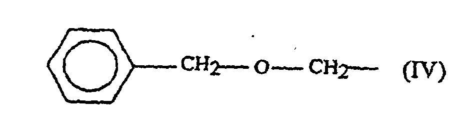 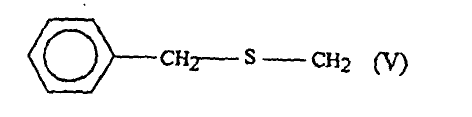
(CH₃)₂-CH-CH₂- (VI)

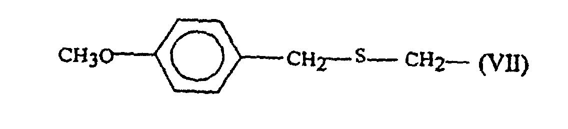

6. Pseudo-peptide according to claim 1, wherein R³ is the group of formula:

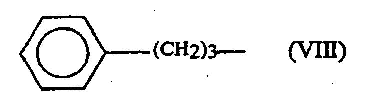

7. Pseudo-peptide according to one of the claims 1 to 6, wherein R⁴ represents the group of formula:

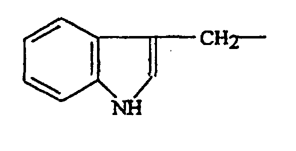

8. Pseudo-peptide according to one of the claims 1 to 6, wherein R¹ represents a group chosen from among the following groups: acetyl, benzyloxyacetyl, phenylaminoacetyl, m-(chlorophenyl)-aminoacetyl, (2-hydroxy-5-chloro-phenyl) aminoacetyl, indolyl-2-carbonyl, 4,6-dichloro-indolyl-2-carbonyl, quinolyl-2-carbonyl and 1-oxa-2,4-dichloro-7-naphthalene carbonyl.

9. Pseudo-peptide according to claim 1 complying with the formula: 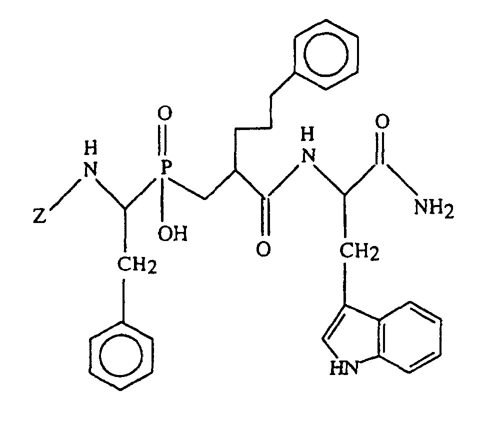 wherein Z is the benzyloxycarbonyl group.

10. Pseudo-peptide according to claim 9, wherein the asymmetrical carbon of the system: 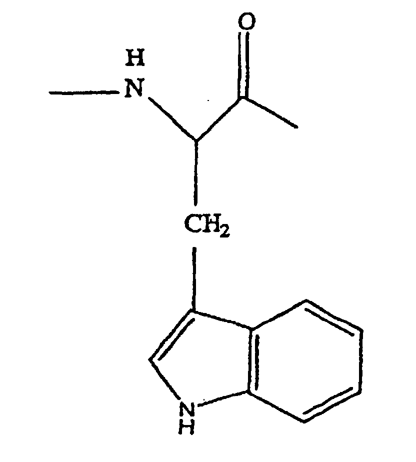 has the L configuration.

11. Pharmaceutical composition for use as an inhibitor of at least one matrix zinc metalloprotease comprising at least one pseudo-peptide according to any one of the claims 1 to 10.

12. Composition according to claim 11, wherein the matrix zinc metalloprotease is chosen from among MMP-2, MMP-8, MMP-9 and MMP-11.

13. Pharmaceutical composition according to either of the claims 11 and 12 for the treatment of a disease **characterized by** the over-expression of matrix proteases.

14. Composition according to claim 13 for the treatment of cancer.

15. Use of a phosphine pseudo-peptide according to one of the claims 1 to 10 for producing a medicament inhibiting at least one matrix zinc metalloprotease.

16. Use according to claim 15, wherein the matrix zinc metalloprotease is chosen from among MMP-2, MMP-8, MMP-9 and MMP-11.

17. Use according to claim 15 or 16, wherein the medicament is intended for the treatment of a disease **characterized by** the over-expression of matrix proteases.

18. Use according to claim 17, wherein the disease is cancer.
